# EUROPEAN PATENT APPLICATION

(11) **EP 4 578 393 A1**
(43) Date of publication of application: **02.07.2025**
(21) Application number: 22956547.8
(22) Date of filing: 26.08.2022
(51) Int. Cl.: A61B 6/00, A61B 6/12

(54) **SURGERY ASSISTANCE DEVICE, ANGIOGRAPHY DEVICE, SURGERY ASSISTANCE SYSTEM, METHOD FOR CONTROLLING SAME, AND COMPUTER PROGRAM**

(71) Applicant: ASAHI INTECC CO., LTD., Seto-shi, Aichi, 489-0071 (JP)
(72) Inventor: KATOH, Osamu, Seto-shi, Aichi 489-0071 (JP); YOSHITAKE, Shumpei, Seto-shi, Aichi 489-0071 (JP); OKAMOTO, Shoma, Seto-shi, Aichi 489-0071 (JP); OWADA, Shun, Seto-shi, Aichi 489-0071 (JP)
(74) Representative: Winter, Brandl - Partnerschaft mbB
(86) International application number: PCT/JP2022/032262
(87) International publication number: WO 2024/042721

(57) **Abstract**

A surgery assistance device includes an angiographic image acquisition unit that sets a time interval corresponding to a pulsation cycle of a heart as predetermined intervals and sequentially acquires an angiographic image representing a target blood vessel into which a medical device is inserted at the predetermined intervals, and an image correction unit that sequentially corrects the angiographic image sequentially acquired by the angiographic image acquisition unit, and that generates a corrected angiographic image by correcting the angiographic image to be corrected in such a manner that a position of a specific portion of the medical device included in the angiographic image to be corrected approaches a position of the specific portion of the medical device included in the angiographic image acquired temporally earlier than the angiographic image to be corrected.

## Description

### TECHNICAL FIELD

The present invention relates to a technique for assisting surgery.

### BACKGROUND ART

In recent years, a flat panel detector (FPD) has been used in blood vessel imaging conducted for examination or treatment. The FPD is a device that has an X-ray tube device and an X-ray flat panel detector and acquires an X-ray image of a blood vessel. An X-ray image acquired by the FPD is also referred to as an "angiographic image", and an imaging apparatus including the FPD is also referred to as an "angiography apparatus". For example, Patent Literature 1 describes that a corrected image and an added corrected image obtained by adding at least one corrected image generated before the corrected image are generated and sequentially displayed, and a stent moving due to pulsation is thereby controlled to be displayed as a fixed moving image in real time.

### CITATION LIST

### Patent Literature

Patent Literature 1: JP 2015-165942 A

### SUMMARY OF INVENTION

### TECHNICAL PROBLEM

Here, like a chronic total occlusion (CTO), the inside of a blood vessel may be obstructed by an obstruction. In such a case, after a medical device is advanced from a true lumen to a false lumen, the obstruction in the blood vessel is removed by a subintimal approach or the like in which the medical device is made to reenter from the false lumen to the true lumen, thereby recanalizing the blood vessel. When performing such a procedure, an operator proceeds with a procedure while checking the position and orientation of the distal end portion of the medical device in the blood vessel while visually recognizing an angiographic image captured by the angiography apparatus.

However, the patient's body is constantly moving due to factors such as the pulsation of the heart and changes in the volume of the thorax associated with breathing. Further, since the medical device is inserted into the body of the patient, the distal end portion of the medical device on the angiographic image is not stationary, and constantly moves with the movement of the body of the patient (in other words, the position of the distal end portion of the medical device on the angiographic image is deviated). There has been a problem that such deviation of the distal end portion of the medical device on the angiographic image makes it difficult for the operator to grasp the position and orientation of the distal end portion of the medical device in the blood vessel. Such a problem is particularly remarkable when the CTO occurs in the coronary artery of the heart which is strongly affected by pulsation. In this regard, Patent Literature 1 describes a technique for controlling the display of a fixed moving image of a stent moving due to pulsation in real time by using an added corrected image, but the technique described in Patent Literature 1 still has room for improvement.

Such a problem is not limited to the recanalization of the CTO, and is common to all examinations and treatments in which a medical device is inserted into a body lumen such as a lymph gland system, a biliary tract system, a urinary tract system, a respiratory tract system, a digestive organ system, a secretory gland, and a genital organ, and a procedure is performed while referring to the medical device photographed in an angiographic image. In addition, such a problem is not limited to the distal end portion of the medical device, and is common to all techniques capable of reducing the positional deviation of a medical device specific portion (a certain specific portion) on the angiographic image.

An object of the present invention is to reduce the positional deviation of a medical device specific portion on an angiographic image.

### SOLUTION TO PROBLEM

The present invention has been made to solve at least part of the above-mentioned problems, and can be practiced as the following aspects.

(1) According to an aspect of the present invention, a surgery assistance device is provided. This surgery assistance device includes an angiographic image acquisition unit that sets a time interval corresponding to a pulsation cycle of a heart as predetermined intervals and sequentially acquires an angiographic image representing a target blood vessel into which a medical device is inserted at the predetermined intervals, and an image correction unit that sequentially corrects the angiographic image sequentially acquired by the angiographic image acquisition unit, and that generates a corrected angiographic image by correcting the angiographic image to be corrected in such a manner that a position of a specific portion of the medical device included in the angiographic image to be corrected approaches a position of the specific portion of the medical device included in the angiographic image acquired temporally earlier than the angiographic image to be corrected.
   According to this configuration, the angiographic image acquisition unit sets a time interval corresponding to a pulsation cycle of a heart as predetermined intervals and sequentially acquires an angiographic image at the predetermined intervals. Since the heart repeats expansion and contraction (hereinafter, also referred to as "expansion/contraction") regularly in accordance with the pulsation cycle, it is possible to acquire angiographic images in which the expansion/contraction states of the heart are equal (uniform) by acquiring an angiographic image at the predetermined intervals corresponding to the pulsation cycle. As a result, the angiographic image acquisition unit can reduce the positional deviation of a medical device specific portion on the angiographic image due to the pulsation. In addition, the body of the patient is constantly moving due to factors other than the pulsation of the heart, such as changes in the volume of the thorax associated with breathing. According to this configuration, in the angiographic images sequentially acquired by the angiographic image acquisition unit, the image correction unit generates a corrected angiographic image by correcting the angiographic image to be corrected in such a manner that a position of a specific portion of the medical device included in the angiographic image to be corrected approaches a position of the specific portion of the medical device included in the angiographic image acquired temporally earlier than the angiographic image to be corrected. Therefore, the image correction unit can reduce the positional deviation of the medical device specific portion on the angiographic image mainly due to a factor other than the pulsation of the heart. As described above, the surgery assistance device according to this aspect separately (individually) performs the reduction of the deviation due to the pulsation by the angiographic image acquisition unit and the reduction of the deviation due to a factor other than the pulsation by the image correction unit. Therefore, it is possible to reduce the positional deviation of the medical device specific portion on the angiographic image with high accuracy. As a result, according to the surgery assistance device of this aspect, since the operator can correctly grasp the position of the medical device specific portion in the target blood vessel, it is possible to improve the accuracy of the procedure, to shorten the time required for the procedure, and to reduce the burden on the patient.
(2) In the surgery assistance device according to the above aspect, the angiographic image acquisition unit may sequentially acquire the angiographic image for which a time interval between two pieces of temporally consecutive electrocardiogram data acquired in the past by an electrocardiogram measurement device is set as the time interval corresponding to the pulsation cycle of the heart. According to this configuration, the predetermined intervals are calculated from the time interval between two pieces of temporally consecutive electrocardiogram data acquired in the past by the electrocardiogram measurement device. Therefore, the angiographic image acquisition unit can acquire an angiographic image in which deviation due to pulsation is accurately reduced in accordance with the pulsation cycle of the heart of each patient.
(3) In the surgery assistance device according to the above aspect, the angiographic image acquisition unit may sequentially acquire the angiographic image for which a time interval between two pieces of temporally consecutive electrocardiogram data acquired immediately before by an electrocardiogram measurement device is set as the time interval corresponding to the pulsation cycle of the heart. According to this configuration, the predetermined intervals are calculated from the time interval between two pieces of temporally consecutive electrocardiogram data acquired immediately before by the electrocardiogram measurement device. Therefore, the angiographic image acquisition unit can acquire an angiographic image in which deviation due to pulsation is accurately reduced in accordance with the pulsation cycle of the heart of each patient, which is the previous pulsation cycle that is close to the current pulsation cycle.
(4) In the surgery assistance device according to the above aspect, the electrocardiogram data is electrocardiogram waveform data. Among the two pieces of consecutive electrocardiogram waveform data, data acquired relatively later is set as n-th electrocardiogram waveform data. When data acquired relatively earlier is defined as (n-1)-th electrocardiogram waveform data, the angiographic image acquisition unit may sequentially acquire the angiographic image for which a time interval between a time point tn-1 at which a specific waveform appears in the (n-1)-th electrocardiogram waveform data and a time point tn at which the specific waveform appears in the n-th electrocardiogram waveform data is set as the time interval corresponding to the pulsation cycle of the heart. According to this configuration, the predetermined intervals are calculated from the time interval between the time point tn-1 at which the specific waveform appears in the (n-1)-th electrocardiogram waveform data and the time point tn at which the specific waveform appears in the n-th electrocardiogram waveform data among the two pieces of temporally consecutive electrocardiogram waveform data acquired by the electrocardiogram measurement device. Therefore, by determining the specific waveform when the expansion/contraction state of the heart is an expansion/contraction state suitable for visually recognizing the medical device (for example, specific waveform = R wave), the angiographic image acquisition unit can acquire an angiographic image when the expansion/contraction state of the heart is an expansion/contraction state suitable for visually recognizing the medical device. As a result, the visibility of the medical device in the angiographic image can be improved.
(5) In the surgery assistance device according to the above aspect, the image correction unit may correct the angiographic image to be corrected in such a manner that a position of the specific portion of the medical device included in the angiographic image to be corrected approaches a position of the specific portion of the medical device included in the corrected angiographic image obtained by correcting the angiographic image acquired immediately before the angiographic image to be corrected. According to this configuration, the angiographic image to be corrected is corrected using the position of the specific portion of the medical device in the corrected angiographic image obtained by correcting the angiographic image acquired immediately before the angiographic image to be corrected. Therefore, the positional shift of the specific portion of the medical device between the latest correction result (the corrected angiographic image K' for the angiographic image K to be corrected) and the immediately preceding correction result (the corrected angiographic image K-1' for the angiographic image K-1 acquired immediately before the angiographic image to be corrected) can be reduced, and a more natural corrected angiographic image can be provided.
(6) The surgery assistance device according to the above aspect may further include an electrocardiogram information acquisition unit that acquires electrocardiogram data from an electrocardiogram measurement device, and a target image acquisition unit that extracts the angiographic image at the predetermined intervals from continuous angiographic images representing the target blood vessel continuously imaged by an FPD at intervals shorter than the predetermined intervals. According to this configuration, the surgery assistance device further includes the target image acquisition unit that extracts an angiographic image at the predetermined intervals, which are a time interval corresponding to the pulsation cycle of the heart, from the continuous angiographic images representing the target blood vessel continuously imaged by the FPD. In this way, the angiography apparatus including the FPD only needs to continuously supply angiographic images of the target blood vessel to the surgery assistance device (in other words, supply the continuous angiographic images), and thus the expandability of the system including the surgery assistance device and the angiography apparatus can be improved.
(7) The surgery assistance device according to the above aspect may further include a true lumen information acquisition unit that acquires three dimensional position information of a true lumen existing in the target blood vessel, a true lumen image generation unit that generates a true lumen image representing the true lumen, and an image composition unit that generates a composite image by compositing the corrected angiographic image and the true lumen image, and outputs the composite image. The true lumen image generation unit may acquire, from the image correction unit, an angiographic image which is obtained by imaging the target blood vessel by an FPD disposed at a freely-selected imaging position and which is the corrected angiographic image for the angiographic image at the predetermined intervals, and generate a true lumen image representing the true lumen at a position and posture corresponding to the corrected angiographic image by using position information of the freely-selected imaging position and three dimensional position information of the true lumen. According to this configuration, the true lumen image generation unit can generate a true lumen image representing a true lumen at the position and posture corresponding to the corrected angiographic image by using the position information of the freely-selected imaging position at which the angiographic image is acquired and the three dimensional position information of the true lumen acquired by the true lumen information acquisition unit. That is, the true lumen image generation unit can generate a true lumen image representing an image of the true lumen on the basis of the three dimensional position information of the true lumen even when the contrast medium does not flow into the target true lumen or when the contrast medium is not flowing in the blood vessel. In addition, since the image composition unit generates the composite image by compositing the corrected angiographic image at the freely-selected imaging position and the true lumen image representing the image of the true lumen and outputs the composite image, the image of the true lumen of the blood vessel can be displayed on the corrected angiographic image. Therefore, by checking the composite image, the operator can proceed with the procedure while checking the positional relation between the medical device on the corrected angiographic image and the true lumen on the true lumen image. As a result, since the operator can correctly grasp the position of the true lumen in the target blood vessel, it is possible to improve the accuracy of the procedure, to shorten the time required for the procedure, and to reduce the burden on the patient.
(8) In the surgery assistance device according to the above aspect, the angiographic image acquisition unit may sequentially acquire a first angiographic image obtained by imaging by the FPD disposed at a first position and a second angiographic image obtained by imaging by the FPD disposed at a second position different from the first position, which are the angiographic image at the predetermined intervals, the image correction unit may sequentially generate a first corrected angiographic image that is the corrected angiographic image for the first angiographic image, and a second corrected angiographic image that is the corrected angiographic image for the second angiographic image, and the true lumen information acquisition unit may acquire three dimensional position information of the true lumen by using an ultrasonic image which is obtained by imaging an inside of the target blood vessel by an ultrasonic sensor and acquired at the predetermined intervals, position information of the first position, the first corrected angiographic image, position information of the second position, and the second corrected angiographic image. According to this configuration, the true lumen information acquisition unit acquires the three dimensional position information of the true lumen by using the first and second corrected angiographic images in which the deviation due to the pulsation of the heart is reduced and the deviation due to a factor other than the pulsation of the heart (such as a change in the volume of the thorax due to breathing) is reduced. Therefore, the three dimensional position information of the true lumen can be acquired with high accuracy as compared with the case of using an angiographic image in which deviation is not reduced. In addition, since the imaging intervals between the ultrasonic image and the first and second corrected angiographic images can be synchronized using the predetermined intervals, the three dimensional position information of the true lumen can be acquired with higher accuracy.
(9) According to an aspect of the present invention, an angiography apparatus is provided. This angiography apparatus includes an FPD having an X-ray tube device and an X-ray flat panel detector, and a target image acquisition unit that acquires an angiographic image representing a target blood vessel by causing the FPD to image the target blood vessel into which a medical device is inserted at predetermined intervals that are a time interval corresponding to a pulsation cycle of a heart, and outputs the acquired image.
   According to this configuration, the target image acquisition unit sets a time interval corresponding to a pulsation cycle of a heart as predetermined intervals, and acquires an angiographic image representing a target blood vessel by imaging the target blood vessel into which a medical device is inserted at the predetermined intervals, and outputs the acquired image. Since the heart repeats expansion/contraction regularly in accordance with the pulsation cycle, it is possible to acquire angiographic images in which the expansion/contraction states of the heart are equal (uniform) by acquiring an angiographic image at the predetermined intervals corresponding to the pulsation cycle. As a result, the target image acquisition unit can reduce the positional deviation of the medical device specific portion on the angiographic image due to the pulsation. As a result, according to the angiography apparatus of this aspect, since the operator can correctly grasp the position of the medical device specific portion in the target blood vessel, it is possible to improve the accuracy of the procedure, to shorten the time required for the procedure, and to reduce the burden on the patient.
(10) The angiography apparatus according to the above aspect may further include an electrocardiogram information acquisition unit that acquires electrocardiogram data from an electrocardiogram measurement device, and the target image acquisition unit may set a time interval between two pieces of temporally consecutive electrocardiogram data acquired in the past by the electrocardiogram information acquisition unit as the predetermined intervals that are the time interval corresponding to the pulsation cycle of the heart. According to this configuration, the predetermined intervals are calculated from the time interval between two pieces of temporally consecutive electrocardiogram data acquired in the past by the electrocardiogram measurement device. Therefore, the target image acquisition unit can output an angiographic image in which deviation due to pulsation is accurately reduced in accordance with the pulsation cycle of the heart of each patient.
(11) The angiography apparatus according to the above aspect may further include an electrocardiogram information acquisition unit that acquires electrocardiogram data from an electrocardiogram measurement device, and the target image acquisition unit may set a time interval between two pieces of temporally consecutive electrocardiogram data acquired immediately before by the electrocardiogram information acquisition unit as the predetermined intervals that are the time interval corresponding to the pulsation cycle of the heart. According to this configuration, the predetermined intervals are calculated from the time interval between two pieces of temporally consecutive electrocardiogram data acquired immediately before by the electrocardiogram measurement device. Therefore, the target image acquisition unit can output an angiographic image in which deviation due to pulsation is accurately reduced in accordance with the pulsation cycle of the heart of each patient, which is the previous pulsation cycle that is close to the current pulsation cycle.
(12) In the angiography apparatus according to the above aspect, the electrocardiogram data is electrocardiogram waveform data. Among the two pieces of consecutive electrocardiogram waveform data, data acquired relatively later is set as n-th electrocardiogram waveform data. When data acquired relatively earlier is defined as (n-1)-th electrocardiogram waveform data, the target image acquisition unit may set a time interval between a time point tn-1 at which a specific waveform appears in the (n-1)-th electrocardiogram waveform data and a time point tn at which the specific waveform appears in the n-th electrocardiogram waveform data as the predetermined intervals that are the time interval corresponding to the pulsation cycle of the heart. According to this configuration, the predetermined intervals are calculated from the time interval between the time point tn-1 at which the specific waveform appears in the (n-1)-th electrocardiogram waveform data and the time point tn at which the specific waveform appears in the n-th electrocardiogram waveform data among the two pieces of temporally consecutive electrocardiogram waveform data acquired by the electrocardiogram measurement device. Therefore, by determining the specific waveform when the expansion/contraction state of the heart is an expansion/contraction state suitable for visually recognizing the medical device (for example, specific waveform = R wave), the target image acquisition unit can output an angiographic image when the expansion/contraction state of the heart is an expansion/contraction state suitable for visually recognizing the medical device. As a result, the visibility of the medical device in the angiographic image can be improved.
(13) According to an aspect of the present invention, a surgery assistance system is provided. This surgery assistance system includes the surgery assistance device according to the above aspect and the angiography apparatus according to the above aspect. The target image acquisition unit of the angiography apparatus sequentially transmits the angiographic image at the predetermined intervals to the surgery assistance device. The angiographic image acquisition unit of the surgery assistance device sequentially acquires the angiographic image at the predetermined intervals from the angiography apparatus. The image correction unit of the surgery assistance device sets a latest angiographic image among a plurality of angiographic images acquired by the angiographic image acquisition unit as the angiographic image to be corrected.
   According to this configuration, the target image acquisition unit of the angiography apparatus sets a time interval corresponding to a pulsation cycle of a heart as predetermined intervals, sequentially transmits the angiographic image at the predetermined intervals to the surgery assistance device, and the angiographic image acquisition unit of the surgery assistance device sequentially acquires the angiographic image at the predetermined intervals from the angiography apparatus, and therefore, the processing load in the system can be distributed between the angiography apparatus and the surgery assistance device, and the occurrence of processing delays due to increased processing load can be suppressed. In addition, since the image correction unit of the surgery assistance device sets the latest angiographic image among the plurality of angiographic images acquired by the angiographic image acquisition unit as the angiographic image to be corrected, it is possible to output the corrected angiographic image for the latest angiographic image without delay.
(14) In the surgery assistance system according to the above aspect, the surgery assistance device may further include a true lumen information acquisition unit that acquires three dimensional position information of a true lumen existing in the target blood vessel, a true lumen image generation unit that generates a true lumen image representing the true lumen, and an image composition unit that generates a composite image by compositing the corrected angiographic image and the true lumen image, and outputs the composite image. The true lumen image generation unit may acquire, from the image correction unit, an angiographic image which is obtained by imaging the target blood vessel by the FPD disposed at a freely-selected imaging position and which is the corrected angiographic image for the angiographic image at the predetermined intervals, and generate a true lumen image representing the true lumen at a position and posture corresponding to the corrected angiographic image by using position information of the freely-selected imaging position and three dimensional position information of the true lumen. According to this configuration, by checking the composite image, the operator can proceed with the procedure while checking the positional relation between the medical device on the corrected angiographic image and the true lumen on the true lumen image. As a result, since the operator can correctly grasp the position of the true lumen in the target blood vessel, it is possible to improve the accuracy of the procedure, to shorten the time required for the procedure, and to reduce the burden on the patient.
(15) In the surgery assistance system according to the above aspect, the angiographic image acquisition unit may sequentially acquire a first angiographic image obtained by imaging by the FPD disposed at a first position and a second angiographic image obtained by imaging by the FPD disposed at a second position different from the first position, which are the angiographic image at the predetermined intervals, the image correction unit may sequentially generate a first corrected angiographic image that is the corrected angiographic image for the first angiographic image, and a second corrected angiographic image that is the corrected angiographic image for the second angiographic image, and the true lumen information acquisition unit may acquire three dimensional position information of the true lumen by using an ultrasonic image which is obtained by imaging an inside of the target blood vessel by an ultrasonic sensor and acquired at the predetermined intervals, position information of the first position, the first corrected angiographic image, position information of the second position, and the second corrected angiographic image. According to this configuration, the three dimensional position information of the true lumen can be acquired with high accuracy as compared with the case of using an angiographic image in which deviation is not reduced. In addition, since the imaging intervals between the ultrasonic image and the first and second corrected angiographic images can be synchronized using the predetermined intervals, the three dimensional position information of the true lumen can be acquired with higher accuracy.

The present invention has been made to solve at least part of the above-mentioned problems, and can be practiced as the following aspects. For example, the present invention can be implemented in the aspect of an angiography apparatus, a surgery assistance device, a server apparatus or robot that implements the functions of these apparatuses, a system including these apparatuses, a computer program that implements the functions of these apparatuses and system, a server apparatus for distributing the computer program, a non-transitory storage medium that stores the computer program, and the like.

### BRIEF DESCRIPTION OF DRAWINGS

Fig. 1 is an explanatory diagram illustrating a configuration of a surgery assistance system.
Fig. 2 is a flowchart illustrating an example of a processing procedure of first processing executed by a target image acquisition unit.
Fig. 3 is a diagram illustrating the first processing and second processing.
Fig. 4 is a flowchart illustrating an example of a processing procedure of the second processing executed by a surgery assistance device.
Figs. 5A and 5B are diagrams illustrating the second processing.
Figs. 6A and 6B are diagrams illustrating the second processing.
Fig. 7 is an explanatory diagram illustrating a configuration of a surgery assistance system of a second embodiment.
Fig. 8 is a diagram illustrating an angiographic image acquisition unit of the second embodiment.
Fig. 9 is a diagram illustrating an example of a method in which a true lumen information acquisition unit acquires three dimensional position information of a true lumen.
Figs. 10A to 10C are diagrams illustrating a true lumen image generation unit and an image composition unit.
Fig. 11 is an explanatory diagram illustrating a configuration of a surgery assistance system of a third embodiment.
Fig. 12 is a flowchart illustrating an example of a processing procedure of the first processing in the third embodiment.
Fig. 13 is an explanatory diagram illustrating a configuration of a surgery assistance system of a fourth embodiment.
Fig. 14 is a flowchart illustrating an example of a processing procedure of the first processing in the fourth embodiment.
Fig. 15 is a diagram illustrating the first processing and the second processing of the fourth embodiment.

### EMBODIMENTS OF THE INVENTION

### <First Embodiment>

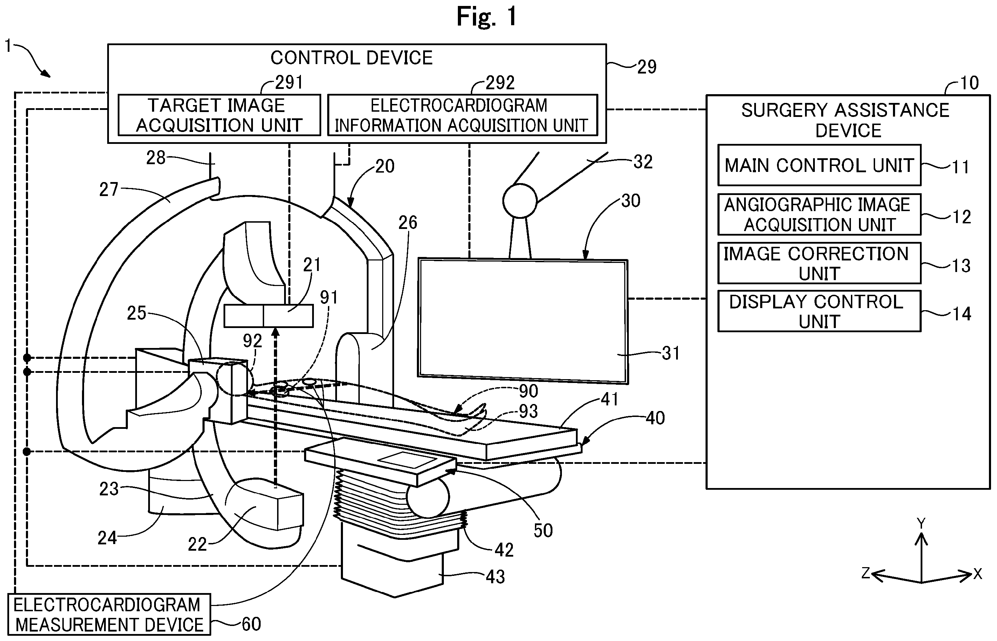

Fig. 1 is an explanatory diagram illustrating a configuration of a surgery assistance system 1. The surgery assistance system 1 is a system that supports examination and treatment. The surgery assistance system 1 includes a surgery assistance device 10, an angiography apparatus 20 having an FPD, a display device 30, a table 40, an operation unit 50, and an electrocardiogram measurement device 60. A captured image of a target blood vessel captured by the FPD is hereinafter also referred to as an "angiographic image".

The surgery assistance system 1 of the present embodiment can acquire an angiographic image in which the positional deviation of a medical device specific portion on the angiographic image due to the pulsation of the heart is reduced by "first processing" described below. Further, the surgery assistance system 1 can generate a corrected angiographic image in which the positional deviation of the medical device specific portion on the angiographic image mainly due to a factor other than the pulsation of the heart is reduced by "second processing" described below. Note that the factor other than the pulsation of the heart is a general term for factors other than the pulsation, such as the movement of the body of the patient in accordance with a change in the volume of the thorax due to breathing and the movement of the body of the patient in accordance with a swallowing motion. In the second processing, as described above, it is possible to reduce deviation due to factors other than the pulsation, but it is also possible to reduce deviation due to the pulsation (that is, deviation due to the pulsation remaining after the first processing).

The "medical device" means any device used for a procedure, such as a guide wire, a penetrating guide wire, a plasma guide wire, a catheter, or an imaging sensor. A "medical device specific portion" means a specific portion of a medical device. The medical device specific portion can be freely determined. For example, the medical device specific portion may be a portion (for example, a distal end portion) of the medical device which is important for examination or treatment, or may be a characteristic portion (for example, a protruding portion, a portion in which a marker is disposed, or the like) of the medical device. The medical device specific portion is also simply referred to as a "specific portion". In the following description, a catheter will be described as an example of the medical device, and a distal end tip of the catheter will be described as an example of the specific portion. Although the term "target blood vessel" means a blood vessel to be targeted for examination or treatment, the surgery assistance system 1 may be used not only for a blood vessel system, but also for a biological lumen such as a lymph gland system, a biliary tract system, a urinary tract system, a respiratory tract system, a digestive organ system, a secretory gland, or a genital organ.

XYZ axes orthogonal to each other are illustrated in Fig. 1. The X-axis corresponds to the width direction of the angiography apparatus 20, the Y-axis corresponds to the height direction of the angiography apparatus 20, and the Z-axis corresponds to the depth direction of the angiography apparatus 20. In the following description, a direction in which a head 92 of a patient (Fig. 1: a human body 90) is present is also simply referred to as a "Z-axis direction" and simply represented as "Z". Three dimensional space formed by three dimensional coordinates (XYZ coordinates) formed by the X, Y, and Z axes is referred to as an XYZ three dimensional space. Note that an origin O of the XYZ three dimensional space (XYZ coordinates) is the position of a heart 91 of the human body 90.

The surgery assistance device 10 is a device that generates and outputs a corrected angiographic image by executing second processing to be described below. The "corrected angiographic image" is an image in which the positional deviation of the medical device specific portion on the angiographic image mainly due to a factor other than the pulsation of the heart is reduced. The surgery assistance device 10 is configured to include a central processing unit (CPU), a read only memory (ROM), and a random access memory (RAM), and the CPU executes a computer program stored in the ROM or the RAM, thereby implementing functions of a main control unit 11, an angiographic image acquisition unit 12, an image correction unit 13, and a display control unit 14. Further, the surgery assistance device 10 includes a storage unit (not illustrated). The storage unit is constituted by a hard disk, a flash memory, a memory card, or the like. The surgery assistance device 10 is electrically connected to each of a control device 29 of the angiography apparatus 20, a display device 30, and the operation unit 50.

The main control unit 11 transmits and receives information to and from the control device 29 of the angiography apparatus 20, the display device 30, and the operation unit 50, and controls the entire surgery assistance device 10.

The angiographic image acquisition unit 12 sets a time interval corresponding to the pulsation cycle of the heart as predetermined intervals and sequentially acquires an angiographic image (in other words, an angiographic image obtained by the first processing) representing the target blood vessel into which the medical device is inserted at the predetermined intervals. The process (step) executed by the angiographic image acquisition unit 12 is also referred to as an angiographic image acquisition process (step).

The image correction unit 13 executes "second processing" that sequentially corrects the angiographic image sequentially acquired by the angiographic image acquisition unit 12. The second processing is processing indicated in the following a1 and a2. Details will be described below. A corrected angiographic image K' is generated by correcting the angiographic image K to be corrected in such a manner that (a1) a position of a specific portion of the medical device included in the angiographic image K approaches (a2) a position of the specific portion of the medical device included in the angiographic image K-x (x is a natural number) acquired temporally earlier than the angiographic image K to be corrected. A process (step) executed by the image correction unit 13 is also referred to as an image correction process (step).

The display control unit 14 causes the display device 30 to output the corrected angiographic image K' generated by the image correction unit 13. A process (step) executed by the display control unit 14 is also referred to as a display control process (step).

The angiography apparatus 20 has an FPD, acquires X-rays transmitted through a human body, and converts the X-rays into a digital signal to acquire an image (angiographic image). The angiography apparatus 20 has a first FPD 21, a first X-ray tube device 22, a first C arm 23, a first support portion 24, a second FPD 25, a second X-ray tube device 26, a second C arm 27, a second support portion 28, and the control device 29.

The first FPD 21 includes an X-ray flat panel detector, converts X-rays entering from the first X-ray tube device 22 into an electrical signal, applies analogue/digital (A/D) conversion, and generates an X-ray image. The first X-ray tube device 22 receives supply of high-voltage power from an X-ray high-voltage apparatus (not illustrated), and irradiates an X-ray beam. As indicated by a bold dashed line in the Y-axis direction in Fig. 1, an X-ray beam irradiated from the first X-ray tube device 22 enters the first FPD 21 via the human body 90. The first C arm 23 is a C-shaped arm (support) that fixes the first FPD 21 and the first X-ray tube device 22 at positions facing each other. The first support portion 24 rotatably supports the first C arm 23. That is, the first FPD 21 and the first X-ray tube device 22 can be moved to a freely-selected imaging position around the human body 90 lying on the bed 41 in a state of being fixed to positions facing each other by the first C arm 23. Hereinafter, the first FPD 21 and the first X-ray tube device 22 fixed to the first C arm 23 is also simply referred to as "first FPD 21".

The configuration of the second FPD 25 is the same as that of the first FPD 21. The configuration of the second X-ray tube device 26 is the same as that of the first X-ray tube device 22. As indicated by a bold dashed line extending in the X-axis direction in Fig. 1, the X-ray beam irradiated from the second X-ray tube device 26 enters the second FPD 25 via the human body 90. The second C arm 27 is a C-shaped arm (support) that fixes the second FPD 25 and the second X-ray tube device 26 at positions facing each other. The second support portion 28 rotatably supports the second C arm 27. That is, the second FPD 25 and the second X-ray tube device 26 can be moved to a freely-selected imaging position around the human body 90 in a state of being fixed to positions facing each other by the second C arm 27. Hereinafter, the second FPD 25 and the second X-ray tube device 26 fixed to the second C arm 27 are simply referred to as the "second FPD 25".

The second FPD 25 is generally arranged in a direction normal to the first FPD 21. For example, as illustrated in Fig. 1, when the first FPD 21 is arranged at an imaging position in a front direction of the human body 90 (a vertical direction of the human body 90 and a longitudinal direction of the human body 90), the second FPD 25 is located at an imaging position in a horizontal direction of the human body 90 (a lateral direction of the human body 90). The angiography apparatus 20 may be simply referred to as an "FPD", an "FPD apparatus", or the like.

The control device 29 includes a CPU, a ROM, and a RAM. The CPU executes a computer program stored in the ROM or the RAM to control the entire angiography apparatus 20. The control device 29 is electrically connected to each of the surgery assistance device 10, the first FPD 21, the second FPD 25, the first support portion 24, the second support portion 28, the display device 30, the table 40, the operation unit 50, and the electrocardiogram measurement device 60. The control device 29 transmits and receives information to and from the surgery assistance device 10, the display device 30, the table 40, the operation unit 50, and the electrocardiogram measurement device 60. The control device 29 drives the first support portion 24 to rotate the first C arm 23 and drives the second support portion 28 to rotate the second C arm 27 in accordance with an operation from the operation unit 50. Further, in accordance with an operation from the operation unit 50, the control device 29 changes the height of the bed 41 by expanding and contracting an expansion/contraction portion 42, and changes the position of the bed 41 by moving the table 40 in the Z-axis direction.

The control device 29 further implements functions of a target image acquisition unit 291 and an electrocardiogram information acquisition unit 292 by the CPU executing a computer program stored in the ROM or the RAM.

The target image acquisition unit 291 performs "first processing" in which a time interval corresponding to the pulsation cycle of the heart is set as predetermined intervals, the target blood vessel is imaged by the first FPD 21 (or the second FPD 25) at the predetermined intervals, and an angiographic image of the target blood vessel acquired by imaging is output to the surgery assistance device 10. Details of the first processing will be described below.

The electrocardiogram information acquisition unit 292 acquires electrocardiogram data measured by the electrocardiogram measurement device 60 from the electrocardiogram measurement device 60. The "electrocardiogram data" of the present embodiment includes both information indicated in the following b1 and b2. The electrocardiogram information acquisition unit 292 may acquire only one of the electrocardiogram waveform data of b1 and the trigger signal of b2 from the electrocardiogram measurement device 60.
(b1) Electrocardiogram waveform data including P wave, Q wave, R wave, S wave, T wave and U wave,
(b2) A trigger signal which becomes 1 (ON) at a timing at which a freely-selected specific waveform (for example, R wave) appears and becomes 0 (OFF) at a timing at which another waveform appears. The electrocardiogram measurement device 60 may be configured to be able to change a specific waveform to be 1 (ON).

The display device 30 is connected to the surgery assistance device 10 and the control device 29 of the angiography apparatus 20, and functions as an output interface for the surgery assistance device 10 and the angiography apparatus 20. The display device 30 has a monitor 31 and an arm 32. The monitor 31 is a "display unit" constituted by a well-known means such as a liquid crystal display, smart glasses, or a projector. The arm 32 supports and fixes the monitor 31.

The table 40 is a table for laying the human body 90 and positioning the human body 90 near the first FPD 21 and the second FPD 25. The table 40 has the bed 41, the expansion/contraction portion 42, and a leg portion 43. The bed 41 includes a mattress on which the human body 90 is laid. The bed 41 is supported by the table 40 so as to be movable in the Z-axis direction. The expansion/contraction portion 42 is configured to be able to change the height of the bed 41 by expanding and contracting in the Y-axis direction. The leg portion 43 supports the bed 41 and the expansion/contraction portion 42. As illustrated by a broken line in Fig. 1, the human body 90 is laid on the bed 41 so as to face upward in a state where the head 92 is placed on the side close to the first FPD 21 and the second FPD 25 and feet 93 are placed on the side far from the first FPD 21 and the second FPD 25. In this way, it is easy to acquire an image of the target blood vessel in the heart 91 by the first FPD 21 and the second FPD 25.

The operation unit 50 is connected to the surgery assistance device 10 and the control device 29 of the angiography apparatus 20, and functions as an output interface for the surgery assistance device 10 and the angiography apparatus 20. The operation unit 50 is an "input unit" constituted by well-known means such as a touch panel, an operation button, an operation lever, an operation switch, a keyboard, a mouse, a voice input unit, and a foot switch. In the illustrated example, the operation unit 50 is fixed to the table 40.

The electrocardiogram measurement device 60 is a device that collects and measures electrocardiogram data obtained by inducing a minute electromotive force generated in association with the activity of the heart 91 by electrodes attached to the human body 90 and amplifying the electromotive force. As described above, the electrocardiogram measurement device 60 transmits both the electrocardiogram waveform data of b1 and the trigger signal of b2 to the angiography apparatus 20.

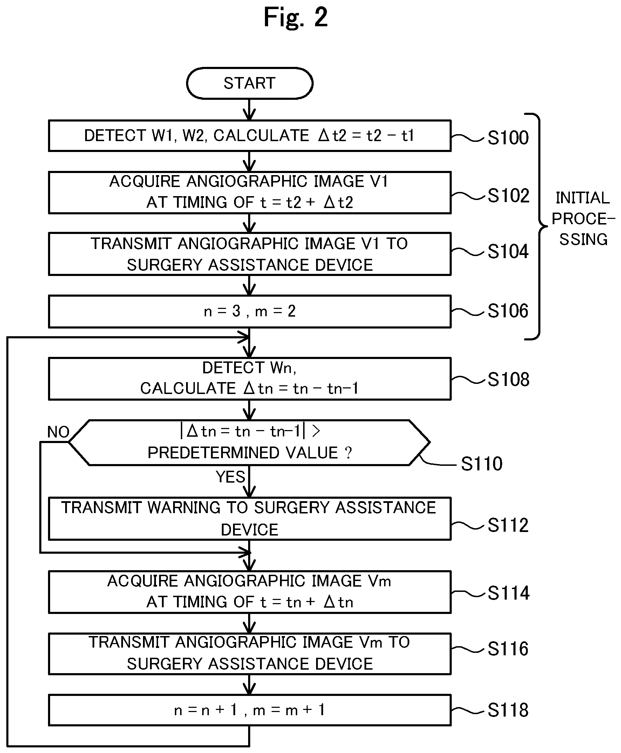

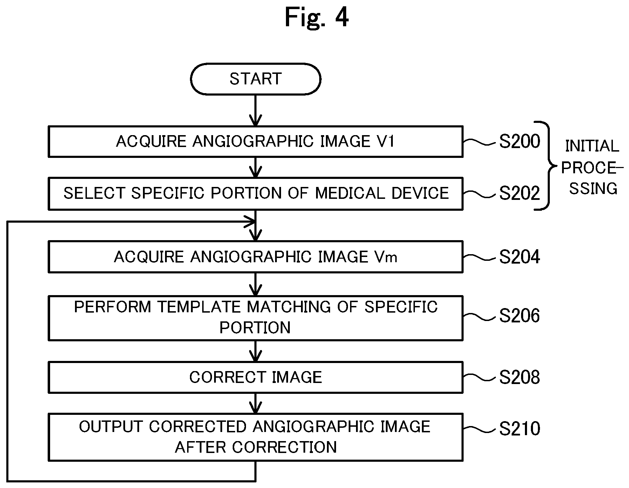

Fig. 2 is a flowchart illustrating an example of a processing procedure of first processing executed by a target image acquisition unit 291. The first processing is processing for setting a time interval corresponding to a pulsation cycle of a heart as predetermined intervals, and acquiring, at the predetermined intervals, an angiographic image representing a target blood vessel by imaging the target blood vessel into which a medical device is inserted. The first processing illustrated in Fig. 2 is started at a freely-selected timing. The freely-selected timing may be, for example, the same time as the power-on of the angiography apparatus 20, the same time as the activation of a predetermined application provided by the control device 29, or the first processing may be started in conjunction with the start of the second processing (to be described below with reference to Fig. 4) in the surgery assistance device 10. In the following description, the FPD (first FPD 21 or second FPD 25) that images a target blood vessel to acquire an angiographic image will be simply referred to as an FPD.

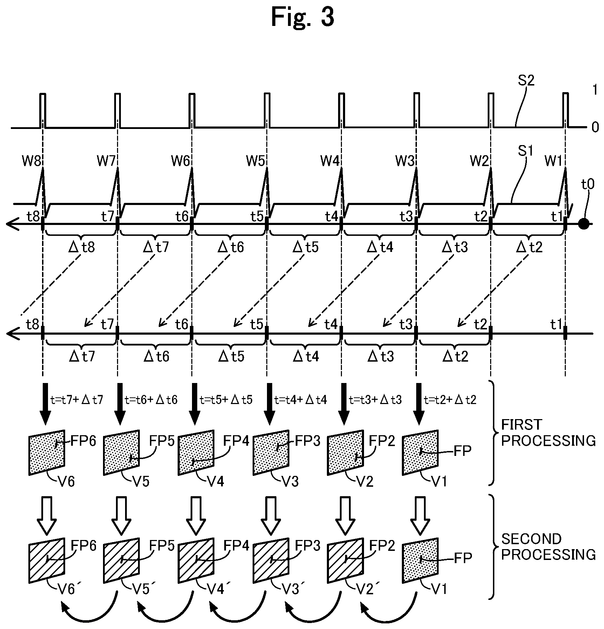

Fig. 3 is a diagram illustrating the first processing and the second processing. In the uppermost part of Fig. 3, a trigger signal S2 of b2 is illustrated in time series in the electrocardiogram data acquired by the control device 29 from the electrocardiogram measurement device 60. Below the trigger signal S2 of Fig. 3, electrocardiogram waveform data S1 of b1 is illustrated in time series in the electrocardiogram data acquired by the control device 29 from the electrocardiogram measurement device 60. A timing at which the trigger signal S2 becomes 1, that is, a timing at which an R wave appears in the electrocardiogram waveform data S1 is also referred to as a "time point Wn (n is a natural number)". In Fig. 3, a broken line extending in the up-down direction on the plane is attached to each time point Wn, and the same time point as each time point Wn is referred to as a "time point tn (n is a natural number)". Below the electrocardiogram waveform data S1 in Fig. 3, the calculation of the predetermined intervals in the control device 29 and a timing t at which the control device 29 causes the FPD to image the target blood vessel to acquire the angiographic image are illustrated along the time series. Below the timing t in Fig. 3, angiographic images Vn (n is a natural number) obtained by imaging by the FPD are illustrated in time series. Below the angiographic image Vn in Fig. 3, corrected angiographic images Vn' corrected by the surgery assistance device 10 through the second processing to be described below are illustrated in time series. Fig. 3 illustrates a case where an interval between Wn and Wn-1 (in other words, an interval between the time point tn and the time point tn-1) is constant. However, in the actual human body 90, the interval between Wn and Wn-1 may be slightly shifted.

Hereinafter, the first processing will be described with reference to Figs. 2 and 3. In step S100 of Fig. 2, the target image acquisition unit 291 detects a time point W1 and a time point W2 with the use of one of the trigger signal S2 and the electrocardiogram waveform data S1 in the electrocardiogram data. When the time points W1 and W2 are detected using the electrocardiogram waveform data S1, the target image acquisition unit 291 may detect the specific waveform (R wave) by performing pattern matching or peak value detection processing on the electrocardiogram waveform data S1. When the trigger signal S2 is used, it is possible to reduce a processing load (processing load for detecting the specific waveform) of the target image acquisition unit 291. After detecting the time points W1 and W2, the target image acquisition unit 291 obtains Δt2 by subtracting the time point t1 corresponding to W1 from the time point t2 corresponding to W2 (Δt2 = t2 - t1).

In step S102, the target image acquisition unit 291 causes the FPD to image the target blood vessel at the timing t (t = t2 + Δt2) after Δt2 obtained in step S100 from the time point t2 corresponding to W2, thereby acquiring an angiographic image V1 of the target blood vessel. In step S104, the target image acquisition unit 291 transmits the acquired angiographic image V1 to the surgery assistance device 10.

In step S106, the target image acquisition unit 291 substitutes 3 for a variable n, substitutes 2 for a variable m (m is a positive integer), and shifts the processing to step S108. Note that steps S100 to S106 are initial processing.

In step S108, the target image acquisition unit 291 detects the time point Wn with the use of one of the trigger signal S2 and the electrocardiogram waveform data S1 in the electrocardiogram data. The target image acquisition unit 291 obtains Δtn by subtracting the time point tn-1 corresponding to Wn-1 from the time point tn corresponding to Wn (Δtn = tn - tn-1).

For example, if n = 3, then tn = t3 and is tn-1 = t2. Therefore, Δt3 = t3 - t2 is obtained. That is, tn of tn and tn-1 is a time point at which the trigger signal S2 = 1 (ON) in the electrocardiogram data acquired relatively later of the two pieces of consecutive electrocardiogram data, and is a time point at which the specific waveform appears in the electrocardiogram waveform data S1 in the electrocardiogram data acquired relatively later. On the other hand, tn-1 of tn and tn-1 is a time point at which the trigger signal S2 = 1 (ON) in the electrocardiogram data acquired relatively earlier of the two pieces of consecutive electrocardiogram data, and is a time point at which the specific waveform appears in the electrocardiogram waveform data S1 in the electrocardiogram data acquired relatively earlier.

In step S110, the target image acquisition unit 291 determines whether an absolute value |Δtn = tn - tn-1| of Δtn obtained in step S108 is larger than a predetermined value. The predetermined value is a threshold value for determining whether the heart 91 of the human body 90 causes arrhythmia or bradycardia, and an appropriate value for determining the presence or absence of arrhythmia or bradycardia is determined in advance and stored in the control device 29. In step S110, the comparison may be performed with respect to both a first threshold value for determining the presence or absence of arrhythmia and a second threshold value for determining the presence or absence of bradycardia. Further, in step S110, it may be determined whether the absolute value |Δtn = tn- tn-1| of Δtn obtained in step S108 is smaller than a third threshold value for determining the presence or absence of tachycardia.

If the absolute value of Δtn is larger than the predetermined value (step S110: YES), the target image acquisition unit 291 transmits a warning to the surgery assistance device 10 in step S112. The surgery assistance device 10 that has received the warning displays a warning message on the display device 30 to call the operator's attention. On the other hand, if the absolute value of Δtn is equal to or smaller than the predetermined value (step S110: NO), the target image acquisition unit 291 shifts the processing to step S114.

In step S114, the target image acquisition unit 291 acquires an angiographic image Vm of the target blood vessel by causing the FPD to image the target blood vessel at the timing t (t = tn + Δtn) after Δtn obtained in step S108 from the time point tn corresponding to Wn. Here, the target image acquisition unit 291 sends an operation command to the FPD slightly before the timing t in consideration of a preparation time required for activation of the FPD, and the like in advance in such a manner that imaging of the FPD (that is, X-ray irradiation by the first X-ray tube device 22 and X-ray detection and conversion by the first FPD 21) is accurately performed at the timing t. This also applies to the above-described step S102.

In step S116, the target image acquisition unit 291 transmits the acquired angiographic image Vm to the surgery assistance device 10.

In step S118, the target image acquisition unit 291 adds 1 to each of the variable n and the variable m, shifts the processing to step S108, and repeats the above-described processing. As a result, the target image acquisition unit 291 of the angiography apparatus 20 sequentially transmits the angiographic images V1, V2, V3, ..., Vm illustrated in Fig. 3 to the surgery assistance device 10. In surgery assistance device 10, the second processing is performed using the angiographic images V1, V2, V3, ..., Vm sequentially acquired from angiography apparatus 20. The processing of Fig. 2 is repeatedly continued until a predetermined termination condition is satisfied. The termination condition may be, for example, the power-off of the angiography apparatus 20 or termination of an application activated at the start of processing.

In this way, in the example of Fig. 3, the angiographic images V1, V2, V3, ..., Vm acquired by the target image acquisition unit 291 of the angiography apparatus 20 by causing the FPD to image the target blood vessel (in other words, the angiographic images V1, V2, V3, ..., Vm acquired by the angiographic image acquisition unit 12 of the surgery assistance device 10) are angiographic images for which a time interval Δtn (Δtn = tn - tn-1) between two pieces of temporally consecutive electrocardiogram data acquired immediately before by the electrocardiogram measurement device 60 is set as predetermined intervals corresponding to the pulsation cycle of the heart, and which are acquired at the predetermined intervals. The heart 91 of the human body 90 repeats expansion/contraction regularly in accordance with the pulsation cycle. Therefore, the target image acquisition unit 291 of the present embodiment predicts that the pulsation cycle of the heart 91 is in the same phase as that at the time point tn-1 (in other words, the expansion/contraction state of the heart 91 is equal to that at the time point tn-1) after Δtn from the time point tn, and causes the FPD to acquire the angiographic image Vm. In the present embodiment, since the specific waveform is the R wave that appears at the end of the diastole, the target image acquisition unit 291 can acquire the angiographic image Vm that captures the heart 91 in a state where the dilation ends, the motion becomes gentle, and the change (motion) of the blood vessel is small.

In the above-described example, the target image acquisition unit 291 causes the FPD to image the target blood vessel at each calculated timing t (t = tn + Δtn) to acquire the angiographic image Vm (still image). Therefore, it is possible to shorten the time for irradiating the human body 90 with X-rays and to reduce the amount of exposure of the human body 90, as compared with a case where the FPD is caused to image the target blood vessel at intervals shorter than Δtn to acquire a moving image of an angiographic image.

Fig. 4 is a flowchart illustrating an example of a processing procedure of the second processing executed by the surgery assistance device 10. As described above, the second processing is the processing indicated in the following a1 and a2. A corrected angiographic image K' is generated by correcting the angiographic image K to be corrected in such a manner that (a1) a position of a specific portion of the medical device included in the angiographic image K to be corrected approaches (a2) a position of the specific portion of the medical device included in the angiographic image K-x (x is a natural number) acquired temporally earlier than the angiographic image K to be corrected.

The second processing illustrated in Fig. 4 is started at a freely-selected timing. The freely-selected timing may be, for example, the same time as the power-on of the surgery assistance device 10, the same time as the activation of a predetermined application provided by the surgery assistance device 10, or the first processing may be started in conjunction with the start of the first processing (Fig. 2) in the angiography apparatus 20.

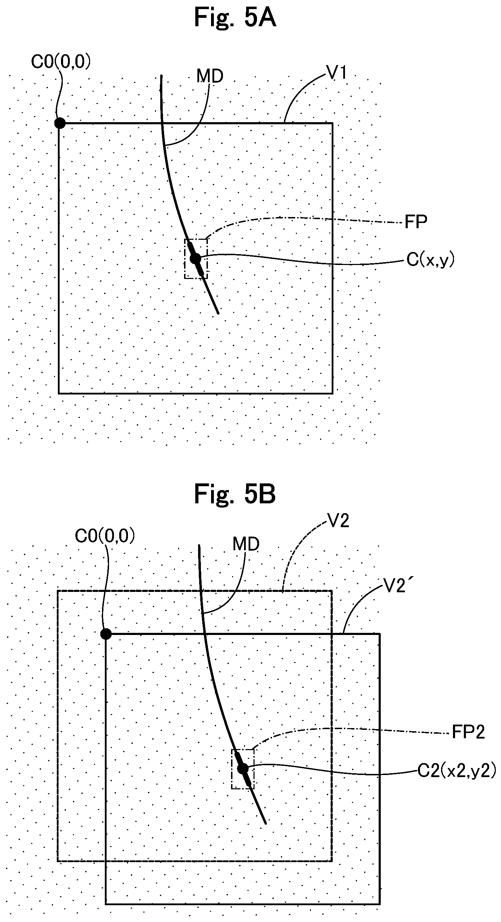

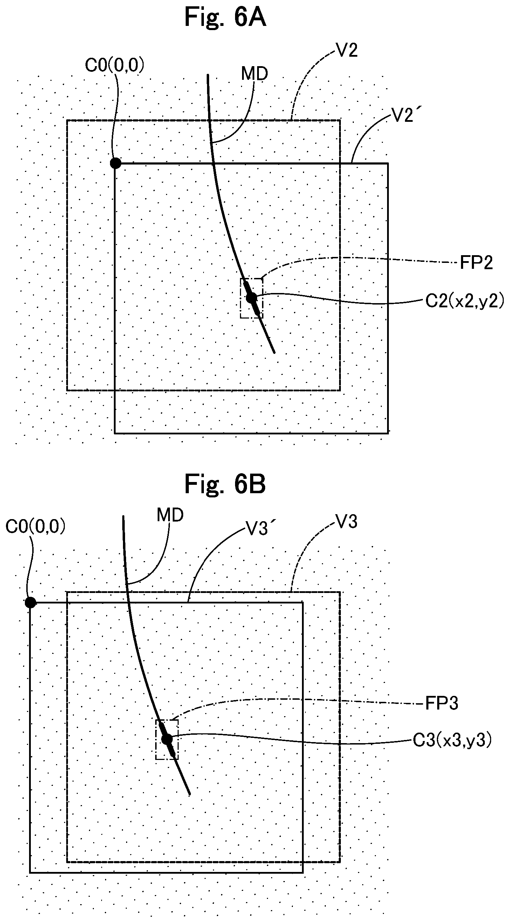

Figs. 5A, 5B, 6A and 6B are diagrams illustrating the second processing. In step S200 of Fig. 4, the angiographic image acquisition unit 12 acquires the angiographic image V1 (the image transmitted in step S104 of Fig. 2) from the angiography apparatus 20. Fig. 5A illustrates an example of the angiographic image V1.

In step S202, the display control unit 14 causes the display device 30 to display the angiographic image V1 acquired in step S200. Thereafter, the image correction unit 13 guides the operator to select a specific portion (a specific portion: a distal end portion, a protruding portion, a marker portion, or the like) of the medical device. The guidance may be performed by displaying characters or an image including a message for prompting the selection on the display device 30, or may be performed by outputting a voice message for prompting the selection from the display device 30. In accordance with this guidance, the operator selects a specific portion of the medical device photographed in the angiographic image V1 displayed on the display device 30. The selection can be performed by selecting a range of a portion corresponding to the specific portion of the medical device in the angiographic image V1 displayed on the display device 30, as indicated by a rectangular frame of a one dot chain line in Fig. 5A. In the example of Fig. 5A, a "distal end tip" of a medical device MD (for example, a catheter) is exemplified as a specific portion FP. The selection may be performed by other well-known means, such as tapping, clicking, etc.

After the specific portion FP of the medical device photographed in the angiographic image V1 is selected, the image correction unit 13 calculates coordinates x, y of the position of the center of gravity of the specific portion FP on the angiographic image V1, and sets the calculated coordinates x, y of the position of the center of gravity as a center C (x, y) of the specific portion FP. The image correction unit 13 extracts an image in a predetermined range including the selected specific portion FP (for example, an image corresponding to the inside of the rectangular frame in Fig. 5A) from the angiographic image V1, and stores the image in a storage unit (not illustrated) together with coordinate information of the center C (x, y) of the specific portion FP. Hereinafter, the stored image is also referred to as a "specific portion image". Note that steps S200 and S202 are initial processing.

In step S204, the angiographic image acquisition unit 12 acquires the angiographic image Vm (Fig. 2: the image transmitted in step S116) from the angiography apparatus 20. Fig. 5B illustrates an example of the angiographic image Vm (m = 2) as an example of the angiographic image Vm acquired temporally later than the angiographic image V1.

In step S206, the image correction unit 13 performs template matching of the specific portion of the medical device by using the angiographic image Vm acquired in step S204. To be specific, the image correction unit 13 detects a specific portion FPm (m = 2) of the medical device photographed in the angiographic image Vm as illustrated in Fig. 5B by matching the image of the medical device photographed in the angiographic image Vm and the image of the specific portion FP of the medical device included in the specific portion image. Further, the image correction unit 13 calculates a center Cm (xm, ym: m = 2) of the specific portion FPm on the angiographic image Vm, that is, coordinates xm, ym of the position of the center of gravity.

In step S208, the image correction unit 13 performs image correction. The image correction is performed in accordance with a1 and a2 described above. Hereinafter, a specific example of image correction will be described with reference to Figs. 5A, 5B, 6A, and 6B. Fig. 6A illustrates an example of the angiographic image Vm (m = 2) as an example of the angiographic image Vm acquired temporally later than the angiographic image V1, and Fig. 6B illustrates an example of the angiographic image Vm (m = 3) as an example of the angiographic image Vm acquired temporally later than the angiographic image V1.

A specific example of Figs. 5A and 5B will be described. In step S208, which is executed for the first time, a corrected angiographic image V2' is generated by correcting the angiographic image V2 to be corrected by moving same in parallel in such a manner that (a1) a position x2, y2 of a specific portion FP2 of the medical device MD included in the angiographic image V2 to be corrected approaches (a2) a position x, y of the specific portion FP of the medical device MD included in the angiographic image V1 acquired temporally earlier than the angiographic image V2 to be corrected. Here, "moving the image in parallel in such a manner that the position x2, y2 approaches the position x, y" means that coordinates (x2, y2) approaches coordinates (x, y), that is, the upper left end C0 (0, 0) of the image is translated in such a manner that the difference between x2 and x becomes small and the difference between y2 and y becomes small. As a result, the position of the medical device MD in the image is changed in accordance with the change in the display range of the image in the angiographic image before and after the correction.

A specific example of Figs. 6A and 6B will be described. In step S208, which is executed for the second time, a corrected angiographic image V3' is generated by correcting by moving the range of the angiographic image V3 to be corrected in parallel in such a manner that (a1) a position x3, y3 of a specific portion FP3 of the medical device MD included in the angiographic image V3 to be corrected approaches (a2') the position x2, y2 of the specific portion FP2 of the medical device MD included in the corrected angiographic image V2' obtained by correcting the angiographic image V2 acquired immediately before the angiographic image V2 to be corrected.

In this way, in the first (initial) image correction, the image correction unit 13 corrects the angiographic image to be corrected with the use of the position of the specific portion FP in the angiographic image V1 serving as a reference. Further, in the second and subsequent image corrections, the image correction unit 13 corrects the angiographic image to be corrected by using the position of the specific portion FP in the "corrected angiographic image after the correction" for the angiographic image acquired immediately before the angiographic image to be corrected, as described in the above a2'.

Returning to Fig. 4, the description will be continued. In step S210 of Fig. 4, the display control unit 14 displays (outputs) the corrected angiographic image Vm' corrected in step S208 on the display device 30. Thereafter, the display control unit 14 shifts the processing to step S204, and repeats the above-described processing. As a result, in the display device 30, after the angiographic image V1 serving as the reference, corrected angiographic images V2', V3', ..., Vm' sequentially acquired from the angiography apparatus 20 by the angiographic image acquisition unit 12 and sequentially corrected by the image correction unit 13 are output to the display device 30 (see the lowermost part of Fig. 3). By referring to the corrected angiographic images V2', V3', ..., Vm' output to the display device 30, the operator can grasp the position of the specific portion of the medical device and proceed with the procedure.

The processing of Fig. 4 is repeatedly continued until a predetermined termination condition is satisfied. The termination condition may be, for example, the power-off of the surgery assistance device 10 or termination of an application activated at the start of processing.

As described above, according to the surgery assistance device 10 of the first embodiment, the angiographic image acquisition unit 12 sequentially acquires the angiographic image Vm at the predetermined intervals Δtn which is the time interval corresponding to the pulsation cycle of the heart 91. Since the heart 91 repeats expansion and contraction (expansion/contraction) regularly in accordance with the pulsation cycle, it is possible to acquire angiographic images Vm in which the expansion/contraction states of the heart 91 are equal (uniform) by acquiring the angiographic image Vm at the predetermined intervals Δtn corresponding to the pulsation cycle. By acquiring such an angiographic image Vm, the angiographic image acquisition unit 12 can reduce the positional deviation of the medical device specific portion on the angiographic image due to the pulsation. In addition, the body of the patient (human body 90) is constantly moving due to factors other than the pulsation of the heart 91, such as changes in the volume of the thorax associated with breathing. According to the surgery assistance device 10 of the first embodiment, the image correction unit 13 generates, in the angiographic images Vm sequentially acquired by the angiographic image acquisition unit 12, a corrected angiographic image V2' by correcting the angiographic image V2 to be corrected in such a manner that a position of the specific portion FP2 of the medical device included in the angiographic image V2 to be corrected approaches a position of the specific portion FP of the medical device included in the angiographic image V1 acquired temporally earlier than the angiographic image V2 to be corrected (Fig. 5). Therefore, the image correction unit 13 can reduce the positional deviation of the medical device specific portion on the angiographic image mainly due to a factor other than the pulsation of the heart 91. As described above, the surgery assistance device 10 according to the first embodiment separately (individually) performs the reduction of the deviation due to the pulsation by the angiographic image acquisition unit 12 and the reduction of the deviation due to a factor other than the pulsation by the image correction unit 13. Therefore, it is possible to reduce the positional deviation of the medical device specific portion on the angiographic image with high accuracy. As a result, according to the surgery assistance device 10 of the first embodiment, since the operator can correctly grasp the position of the medical device specific portion in the target blood vessel, it is possible to improve the accuracy of the procedure, to shorten the time required for the procedure, and to reduce the burden on the patient.

Further, according to the surgery assistance device 10 of the first embodiment, the image correction unit 13 corrects the angiographic image V3 to be corrected by using the position of the specific portion FP2 of the medical device in the corrected angiographic image V2' obtained by correcting the angiographic image V2 acquired immediately before the angiographic image V3 to be corrected (Fig. 6). Therefore, the positional shift of the specific portion of the medical device between the latest correction result (the corrected angiographic image K' = V3' for the angiographic image K = V3 to be corrected) and the immediately preceding correction result (the corrected angiographic image K-1' = V2' for the angiographic image K-1 = V2 acquired immediately before the angiographic image to be corrected) can be reduced, and a more natural corrected angiographic image can be provided.

Further, according to the angiography apparatus 20 of the first embodiment, the target image acquisition unit 291 acquires the angiographic image Vm representing a target blood vessel by causing the FPD to image the target blood vessel into which a medical device is inserted at the predetermined intervals Δtn that are the time interval corresponding to the pulsation cycle of the heart 91, and outputs the acquired image to the surgery assistance device 10. Since the heart 91 repeats expansion/contraction regularly in accordance with the pulsation cycle, it is possible to acquire angiographic images Vm in which the expansion/contraction states of the heart 91 are equal (uniform) by acquiring the angiographic image Vm at the predetermined intervals Δtn corresponding to the pulsation cycle. As a result, the target image acquisition unit 291 can reduce the positional deviation of the medical device specific portion on the angiographic image due to the pulsation. As a result, according to the angiography apparatus 20 of the first embodiment, since the operator can correctly grasp the position of the medical device specific portion in the target blood vessel, it is possible to improve the accuracy of the procedure, to shorten the time required for the procedure, and to reduce the burden on the patient.

Further, according to the angiography apparatus 20 of the first embodiment, the predetermined intervals Δtn are calculated from the time interval tn - tn-1 between the two pieces of temporally consecutive electrocardiogram data acquired immediately before by the electrocardiogram measurement device 60 (Fig. 3). Therefore, the target image acquisition unit 291 can acquire by imaging and output the angiographic image Vm in which deviation due to pulsation is accurately reduced in accordance with the pulsation cycle of the heart 91 of each patient, which is the previous pulsation cycle that is close to the current pulsation cycle. Similarly, the angiographic image acquisition unit 12 of the surgery assistance device 10 can acquire the angiographic image Vm in which deviation due to pulsation is accurately reduced.

Further, according to the angiography apparatus 20 of the first embodiment, the predetermined intervals Δtn are calculated from the time interval tn - tn-1 between the time point tn-1 at which the specific waveform appears in the (n-1)-th electrocardiogram waveform data and the time point tn at which the specific waveform appears in the n-th electrocardiogram waveform data among the two pieces of temporally consecutive electrocardiogram waveform data S1 acquired by the electrocardiogram measurement device 60 (Fig. 3). Therefore, by determining the specific waveform when the expansion/contraction state of the heart 91 is an expansion/contraction state suitable for visually recognizing the medical device (for example, the specific waveform = R wave), the target image acquisition unit 291 can acquire by imaging and output the angiographic image Vm when the expansion/contraction state of the heart 91 is an expansion/contraction state suitable for visually recognizing the medical device. As a result, the visibility of the medical device in the angiographic image can be improved. Similarly, the angiographic image acquisition unit 12 of the surgery assistance device 10 can acquire the angiographic image Vm when the expansion/contraction state of the heart 91 is an expansion/contraction state suitable for visually recognizing the medical device.

Further, according to the surgery assistance system 1 of the first embodiment, the target image acquisition unit 291 of the angiography apparatus 20 sequentially transmits the angiographic image Vm at the predetermined intervals Δtn that are the time interval corresponding to the pulsation cycle of the heart 91 to the surgery assistance device 10 (Fig. 2: step S116), and the angiographic image acquisition unit 12 of the surgery assistance device 10 sequentially acquires the angiographic image Vm at the predetermined intervals Δtn from the angiography apparatus 20 (Fig. 4: step S204). Therefore, the processing load in the surgery assistance system 1 can be distributed between the angiography apparatus 20 and the surgery assistance device 10, and the occurrence of processing delays due to increased processing load can be suppressed. Furthermore, since the image correction unit 13 of the surgery assistance device 10 sets the latest angiographic image among the plurality of angiographic images acquired by the angiographic image acquisition unit 12 as the angiographic image to be corrected (Fig. 4: step S204), it is possible to output the corrected angiographic image Vm' for the latest angiographic image Vm without delay (Fig. 4: step S210).

### <Second Embodiment>

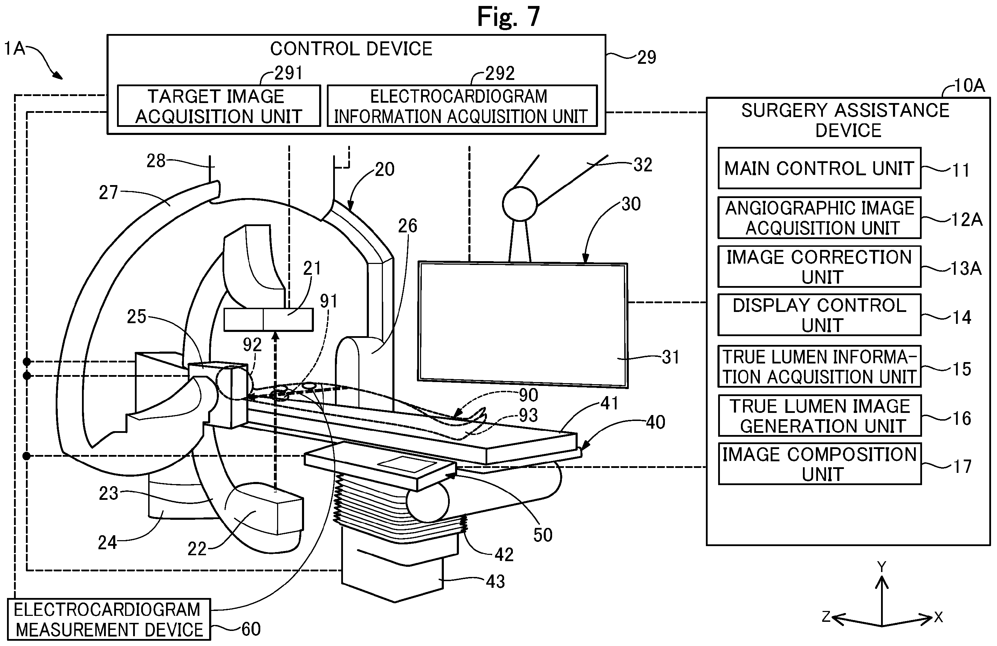

Fig. 7 is an explanatory diagram illustrating a configuration of a surgery assistance system 1A of a second embodiment. In the second embodiment, a configuration will be described in which, for a corrected angiographic image of a target blood vessel, a true lumen image representing a true lumen in the target blood vessel having a position and posture corresponding to the corrected angiographic image is generated, and a composite image obtained by compositing the corrected angiographic image and the true lumen image is displayed. The surgery assistance system 1A of the second embodiment includes a surgery assistance device 10A instead of the surgery assistance device 10. In the configuration described in the first embodiment, the surgery assistance device 10A includes an angiographic image acquisition unit 12A instead of the angiographic image acquisition unit 12, an image correction unit 13A instead of the image correction unit 13, and further includes a true lumen information acquisition unit 15, a true lumen image generation unit 16, and an image composition unit 17.

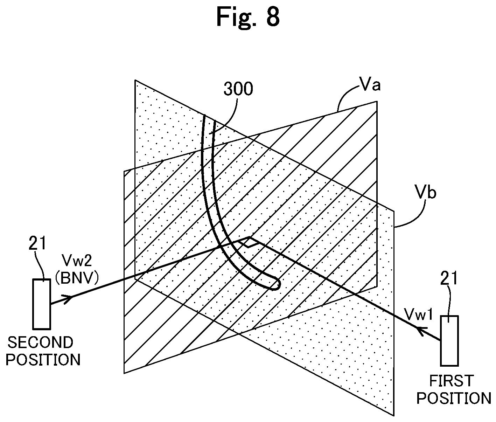

Fig. 8 is a diagram illustrating an angiographic image acquisition unit 12A of the second embodiment. The angiographic image acquisition unit 12A acquires a first angiographic image Va imaged by the first FPD 21 disposed at a first position and a second angiographic image Vb imaged by the first FPD 21 disposed at a second position different from the first position, which are angiographic images at the predetermined intervals Δtn, by the first processing described with reference to Fig. 2. The first angiographic image Va and the second angiographic image Vb are the same as the angiographic images V1, V2 of the first embodiment in that they are images in which the pulsation cycle of the heart 91 is in the same phase, that is, images in which the expansion/contraction states of the heart 91 are the same.

Meanwhile, the first angiographic image Va and the second angiographic image Vb are different from each other in the position of the first FPD 21 where the images Va, Vb are imaged. To be specific, as illustrated in Fig. 8, the second angiographic image Vb (Fig. 8: dot hatching) is an image acquired by the first FPD 21 disposed in the imaging direction perpendicular to the first angiographic image Va (Fig. 8: oblique hatching). In Fig. 8, a vector representing a first view which is an imaging direction with respect to the heart 91 of the first FPD 21 disposed at the first position is represented as a first view vector Vw1, and a vector representing a second view (= BNV) which is an imaging direction with respect to the heart 91 of the first FPD 21 disposed at the second position is represented as a second view vector Vw2.

The image correction unit 13A generates a first corrected angiographic image Va', which is a corrected angiographic image for the first angiographic image Va, and a second corrected angiographic image Vb', which is a corrected angiographic image for the second angiographic image Vb, respectively, by the second processing described with reference to Fig. 4, and transmits same to the true lumen information acquisition unit 15.

The true lumen information acquisition unit 15 acquires three dimensional position information of the true lumen existing in the target blood vessel with the use of the angiographic image (specifically, the first corrected angiographic image Va' and the second corrected angiographic image Vb') acquired from the image correction unit 13A. The process (step) executed by the true lumen information acquisition unit 15 is also referred to as a true lumen information acquisition process (step).

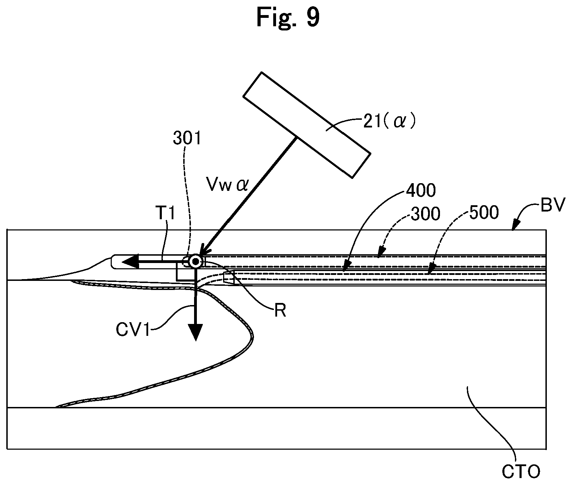

Fig. 9 is a diagram illustrating an example of a method in which the true lumen information acquisition unit 15 acquires the three dimensional position information of the true lumen. Fig. 9 is a longitudinal section of a target blood vessel BV in which a CTO has occurred, and illustrates an example in which the imaging sensor 300 (ultrasonic sensor 300) as a medical device and the wire catheter 400 into which the guide wire 500 has been inserted are inserted into a false lumen formed under the intima of the target blood vessel BV. In this example, a method will be described in which the true lumen information acquisition unit 15 acquires the three dimensional position information of the true lumen by using position information of the first position (Fig. 8), the first corrected angiographic image Va', position information of the second position (Fig. 8), the second corrected angiographic image Vb', and an ultrasonic image. Here, the first corrected angiographic image Va' and the second corrected angiographic image Vb' include an image of the imaging sensor 300. The ultrasonic image includes an image of the guide wire 500 and an image of the true lumen.

The true lumen information acquisition unit 15 acquires the three dimensional position information of the true lumen by the following procedures c1 to c 11.
(c1) A position vector of the transducer 301 of the imaging sensor 300 and an axis vector T1 of the transducer 301 are obtained using the position information of the first position and the first corrected angiographic image Va' at the first position, and the position information of the second position, and the second corrected angiographic image Vb' at the second position.
(c2) A position α of the first FPD 21 at which the corrected angiographic image Vα' in which the transducer 301 of the imaging sensor 300 and the guide wire 500 overlap (intersect) is obtained is determined, and a view vector Vwα is calculated from the position α. The corrected angiographic image Vα' is a corrected angiographic image generated by the image correction unit 13A through the second processing described with reference to Fig. 4 for the angiographic image Vα at the predetermined intervals Δtn acquired by the angiographic image acquisition unit 12A through the first processing.
(c3) A rotation axis R is calculated from the outer product of the axis vector T1 of the transducer 301 calculated in procedure c1 and the view vector Vwα calculated in procedure c2.
(c4) A vector CV1 obtained by rotating the axis vector T1 of the transducer 301 calculated in procedure c1 by 90° about the rotation axis R calculated in procedure c3 is calculated.
(c5) The imaging sensor 300 is caused to acquire an ultrasonic image of the inside of the target blood vessel in a direction perpendicular to the axis vector T1 of the transducer 301 (360° all circumferential directions of T1) by imaging. At this time, the ultrasonic image is acquired at the same timing t as the timing t at which the first angiographic image Va, the second angiographic image Vb, and the angiographic image Vα are imaged (the timing t at the predetermined intervals which are the time interval corresponding to the pulsation cycle of the heart).
(c6) Association in the direction from the transducer 301 to the guide wire 500 is performed using the ultrasonic image obtained in the procedure c5.
(c7) The number of pixels in the ultrasonic image is associated with the actual dimensions.
(c8) A true lumen vector S1 (a vector which is perpendicular to the transducer axis T1 and extends from the transducer 301 to the true lumen) in the XYZ three dimensional space is calculated using the image of the guide wire 500 photographed in the ultrasonic image and the image of the true lumen.
(c9) The actual width and length of the true lumen is calculated using the true lumen vector S1 and the associated dimensions.
(c10) The above processing is repeated by the target number of marks i (i is a natural integer) to calculate the true lumen vectors S1 to Si and the width and length thereof in the XYZ three dimensional space.
(c11) The directions of the obtained true lumen vectors S1 to Si, the lengths (mm) of the true lumen vectors S1 to Si, and the actual dimensions (mm) of the widths of the true lumen corresponding to the true lumen vectors S1 to Si is stored as the three dimensional position information of the true lumen (position information in the XYZ three dimensional space).

An example of another method in which the true lumen information acquisition unit 15 acquires the three dimensional position information of the true lumen will be described. In this example, a method will be described in which the imaging sensor 300 is not used, and the true lumen information acquisition unit 15 acquires three dimensional position information of the true lumen by using the first corrected angiographic image Va' and the second corrected angiographic image Vb', which are images of the true lumen captured from different angles. Here, the first corrected angiographic image Va' includes an image of the distal end portion of the wire catheter 400 and an image of the true lumen. The second corrected angiographic image Vb' includes an image of the distal end portion of the wire catheter 400 and an image of the true lumen. The true lumen information acquisition unit 15 obtains the true lumen vectors S1 to Si by using the images of the true lumen respectively photographed in the first corrected angiographic image Va' and the second corrected angiographic image Vb'.

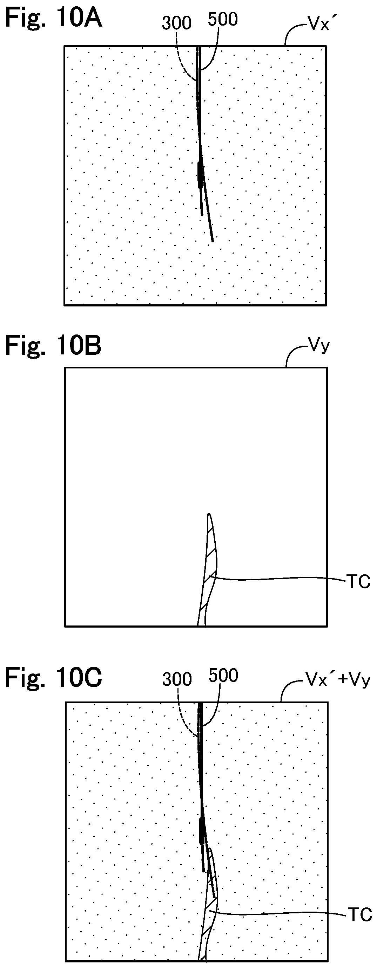

Figs. 10A to 10C are diagrams illustrating the true lumen image generation unit 16 and the image composition unit 17. Fig. 10A illustrates an example of a corrected angiographic image Vx' acquired from the image correction unit 13A. Fig. 10B illustrates an example of a true lumen image Vy generated by the true lumen image generation unit 16. Fig. 10C illustrates an example of a composite image Vx' + Vy generated by the image composition unit 17.

The true lumen image generation unit 16 generates a true lumen image representing a true lumen. To be specific, first, the true lumen image generation unit 16 acquires, from the image correction unit 13, the corrected angiographic image Vx' which is obtained by imaging the target blood vessel BV by the first FPD 21 disposed at a freely-selected imaging position A and which is the corrected angiographic image Vx' for the angiographic image Vx at the predetermined intervals Δtn. Fig. 10A illustrates an example of the corrected angiographic image Vx' acquired by the true lumen image generation unit 16. The imaging position A is a freely-selected position different from the first position, the second position, and the position α described above. Thereafter, the true lumen image generation unit 16 generates a true lumen image Vy representing a true lumen at the position and posture corresponding to the corrected angiographic image Vx' by using an orthogonal projection vector obtained by the position information of the freely-selected imaging position A and the three dimensional position information of the true lumen acquired by the true lumen information acquisition unit 15. As illustrated in Fig. 10B, the true lumen image Vy includes an image of a true lumen TC at the position and posture corresponding to the corrected angiographic image Vx' (in other words, when viewed from the imaging position A). The process (step) executed by the true lumen image generation unit 16 is also referred to as a true lumen image generation process (step).

The image composition unit 17 generates a composite image Vx' + Vy by compositing the corrected angiographic image Vx' illustrated in Fig. 10A and the true lumen image Vy illustrated in Fig. 10B, and outputs the composite image to the display device 30. As illustrated in Fig. 10C, the composite image Vx' + Vy is an image in which the true lumen image Vy is superimposed and displayed on the corrected angiographic image Vx'. The process (step) executed by the image composition unit 17 is also referred to as an image composition process (step).

Details of processing in the true lumen information acquisition unit 15, the true lumen image generation unit 16, and the image composition unit 17 are disclosed in International Application PCT/JP2022/28524.

In this way, further processing may be performed using the corrected angiographic images Va', Vb', Vx' corrected by the image correction unit 13A. As described in the first embodiment, in the corrected angiographic images Va', Vb', Vx', a reduction in deviation due to pulsation and a reduction in deviation due to factors other than pulsation are achieved. By performing further processing using such corrected angiographic images Va', Vb', Vx', the accuracy of subsequent processing can be improved. According to the surgery assistance device 10A and the surgery assistance system 1A of the second embodiment as described above, it is also possible to exhibit the same effects as those of the first embodiment described above.

Further, according to the surgery assistance device 10A of the second embodiment, the true lumen image generation unit 16 can generate the true lumen image Vy representing the true lumen TC at the position and posture corresponding to the corrected angiographic image Vx' by using the position information of the freely-selected imaging position A at which the angiographic image Vx is acquired and the three dimensional position information of the true lumen acquired by the true lumen information acquisition unit 15. That is, the true lumen image generation unit 16 can generate a true lumen image Vy representing an image of the true lumen TC on the basis of the three dimensional position information of the true lumen even when the contrast medium does not flow into the target true lumen or when the contrast medium is not flowing in the blood vessel. In addition, since the image composition unit 17 generates the composite image Vx' + Vy by compositing the corrected angiographic image Vx' at the freely-selected imaging position A and the true lumen image Vy representing the image of the true lumen TC and outputs the composite image Vx' + Vy, the image of the true lumen Vy of the blood vessel can be displayed on the corrected angiographic image Vx'. Therefore, by checking the composite image Vx' + Vy, the operator can proceed with the procedure while checking the positional relation between the medical device 300, 500 on the corrected angiographic image Vx' and the true lumen TC on the true lumen image Vy. As a result, since the operator can correctly grasp the position of the true lumen in the target blood vessel, it is possible to improve the accuracy of the procedure, to shorten the time required for the procedure, and to reduce the burden on the patient.

Further, according to the surgery assistance device 10A of the second embodiment, the true lumen information acquisition unit 15 acquires the three dimensional position information of the true lumen by using the first and second corrected angiographic images Va', Vb' in which the deviation due to the pulsation of the heart 91 is reduced and the deviation due to a factor other than the pulsation of the heart 91 (such as a change in the volume of the thorax due to breathing) is reduced. Therefore, the three dimensional position information of the true lumen can be acquired with high accuracy as compared with the case of using an angiographic image in which deviation is not reduced. In addition, since the imaging interval between the ultrasonic image and the first and second corrected angiographic images Va', Vb' can be synchronized using the predetermined intervals Δtn, the three dimensional position information of the true lumen can be acquired with higher accuracy.

### <Third Embodiment>

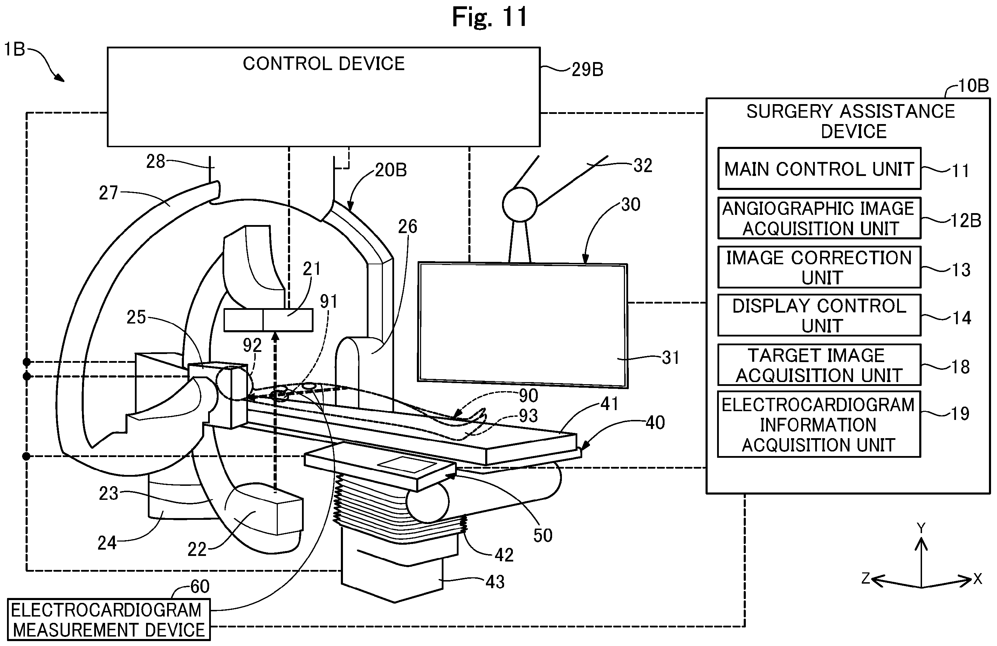

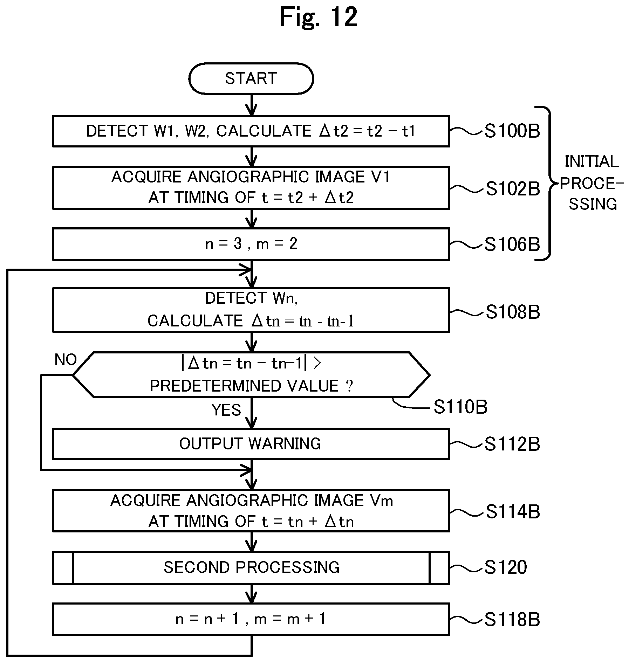

Fig. 11 is an explanatory diagram illustrating a configuration of a surgery assistance system 1B of a third embodiment. In the third embodiment, a configuration in which the surgery assistance device 10B performs the first processing in addition to the second processing will be described. The surgery assistance system 1B of the third embodiment includes a surgery assistance device 10B instead of the surgery assistance device 10, and includes an angiography apparatus 20B instead of the angiography apparatus 20. In the configuration described in the first embodiment, the surgery assistance device 10B includes an angiographic image acquisition unit 12B instead of the angiographic image acquisition unit 12 and further includes a target image acquisition unit 18 and an electrocardiogram information acquisition unit 19. Similarly to the target image acquisition unit 291 of the first embodiment, the target image acquisition unit 18 executes the first processing (described below with reference to Fig. 12). The surgery assistance device 10B is electrically connected to the electrocardiogram measurement device 60, and the electrocardiogram information acquisition unit 19 has the same function as that of the electrocardiogram information acquisition unit 292 of the first embodiment.

The angiography apparatus 20B includes a control device 29B instead of the control device 29 in the configuration described in the first embodiment. The angiography apparatus 20B is not connected to the electrocardiogram measurement device 60. The control device 29B of the angiography apparatus 20B does not have the functions of the target image acquisition unit 291 and the electrocardiogram information acquisition unit 292 described in the first embodiment, and causes the FPD to continuously image a target blood vessel at intervals shorter than the predetermined intervals Δtn described in the first embodiment and transmits the continuous angiographic images acquired by imaging to the surgery assistance device 10B. The "continuous angiographic images" mean a moving image or a smoothly continuous still image at intervals shorter than the predetermined intervals Δtn described in the first embodiment.

Fig. 12 is a flowchart illustrating an example of a processing procedure of the first processing in the third embodiment. The first processing of the third embodiment is executed in the surgery assistance device 10B. The first processing illustrated in Fig. 12 is started at a freely-selected timing. The freely-selected timing may be, for example, the same time as the power-on of the surgery assistance device 10B, or the same time as the activation of a predetermined application provided by the surgery assistance device 10B.

In step S100B, the target image acquisition unit 18 detects the time point W1 and the time point W2 with the use of one of the trigger signal S2 and the electrocardiogram waveform data S1 in the electrocardiogram data acquired by the electrocardiogram information acquisition unit 19, and obtains Δt2. The details are the same as those in step S100 of Fig. 2. In step S102B, the target image acquisition unit 18 extracts the angiographic image V1 at the timing t (t = t2 + Δt2) after Δt2 from the time t2 from the continuous angiographic images received from the angiography apparatus 20B. In step S106B, the target image acquisition unit 18 substitutes 3 for the variable n, and substitutes 2 for the variable m.

In step S108B, the target image acquisition unit 18 detects the time point W1 and the time point Wn with the use of one of the trigger signal S2 and the electrocardiogram waveform data S1 in the electrocardiogram data acquired by the electrocardiogram information acquisition unit 19, and obtains Δtn. The details are the same as those in step S108 of Fig. 2. In step S110B, the target image acquisition unit 18 determines whether the absolute value of Δtn is larger than a predetermined value. If the absolute value of Δtn is larger than the predetermined value (step S110B: YES), the target image acquisition unit 18 causes the display device 30 to output a warning in step S112B, and if the absolute value of Δtn is equal to or smaller than the predetermined value (step S110B: NO), the target image acquisition unit 18 shifts the processing to step S114B. The details are the same as those in step S110 to S112 of Fig. 2.

In step S114B, the target image acquisition unit 18 extracts the angiographic image Vm at the timing t (t = tn + Δtn) after Δtn from the time tn from the continuous angiographic images received from the angiography apparatus 20B. In step S120, the angiographic image acquisition unit 12 acquires the angiographic image Vm extracted by the target image acquisition unit 18, and executes the second processing described with reference to Fig. 4. As described above, in the third embodiment, the second processing is executed as a subroutine of the first processing. In step S118B, the target image acquisition unit 18 adds 1 to each of the variable n and the variable m, shifts the processing to step S108B, and repeats the above-described processing. The processing of Fig. 12 is repeatedly continued until a predetermined termination condition is satisfied. The termination condition may be, for example, the power-off of the surgery assistance device 10B or termination of an application activated at the start of processing.

As described above, the configuration of the surgery assistance system 1B can be variously changed, and the angiography apparatus 20B may be configured to simply continuously image the target blood vessel to acquire the continuous angiographic images, and the surgery assistance device 10B may be configured to execute the first processing of sequentially acquiring the angiographic image Vm at the predetermined intervals Δtn, which are the time interval corresponding to the pulsation cycle of the heart 91. Even in the surgery assistance system 1B of the third embodiment as described above, it is also possible to exhibit the same effects as those of the first embodiment described above. Further, according to the surgery assistance system 1B of the third embodiment, the surgery assistance device 10B further includes the target image acquisition unit 18 that extracts the angiographic image Vm at the predetermined intervals Δtn, which are the time interval corresponding to the pulsation cycle of the heart 91, from the angiographic images representing the target blood vessel continuously imaged by the FPD (continuous angiographic images). In this way, the angiography apparatus 20B including the FPD only needs to continuously supply angiographic images of the target blood vessel to the surgery assistance device 10B (in other words, supply continuous angiographic images), and thus the expandability of the surgery assistance system 1B including the surgery assistance device 10B and the angiography apparatus 20B can be improved.

In steps S102B, S114B of Fig. 12, the target image acquisition unit 18 may transmit an "FPD imaging command" to the control device 29B of the angiography apparatus 20B instead of extracting the angiographic images V1, Vm at the timing t from the continuous angiographic images. It is preferable that the FPD imaging command be transmitted slightly before the timing t in consideration of transmission and reception of the command, a preparation time required for activation of the FPD, and the like in advance in such a manner that imaging of the FPD (that is, X-ray irradiation by the first X-ray tube device 22 and X-ray detection and conversion by the first FPD 21) is accurately performed at the timing t. The control device 29B that has received the FPD imaging command drives the FPD to image the target blood vessel at the timing t to acquire the angiographic images V1, Vm, and transmits the acquired angiographic images V1, Vm to the surgery assistance device 10B. Even in this case, it is possible to construct a system having the same function as that of the third embodiment.

### <Fourth Embodiment>

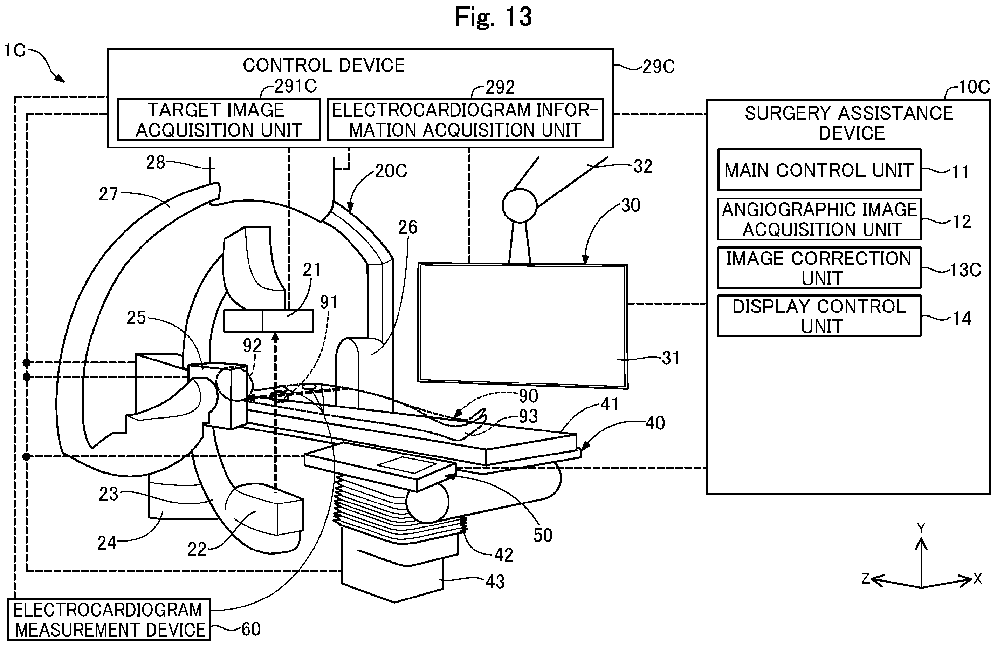

Fig. 13 is an explanatory diagram illustrating a configuration of a surgery assistance system 1C of a fourth embodiment. In the fourth embodiment, a configuration in which the processing contents of the first processing and the second processing are partially different from those of the first embodiment will be described. The surgery assistance system 1C of the fourth embodiment includes a surgery assistance device 10C instead of the surgery assistance device 10, and includes an angiography apparatus 20C instead of the angiography apparatus 20. The surgery assistance device 10C includes an image correction unit 13C instead of the image correction unit 13 in the configuration described in the first embodiment. The angiography apparatus 20C includes a target image acquisition unit 291C instead of the target image acquisition unit 291 in the configuration described in the first embodiment.

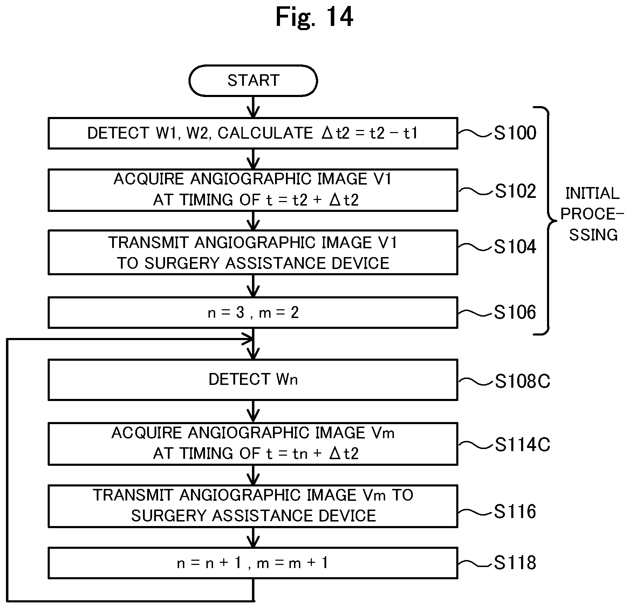

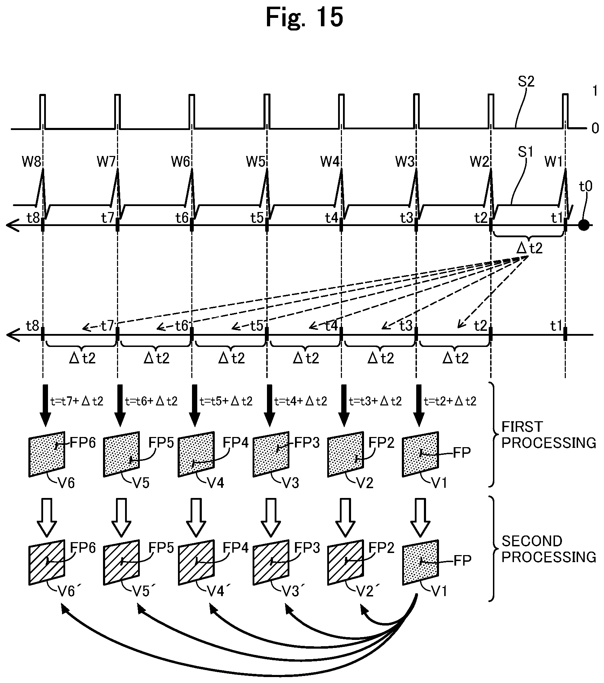

Fig. 14 is a flowchart illustrating an example of a processing procedure of the first processing in the fourth embodiment. Fig. 15 is a diagram illustrating the first processing and the second processing of the fourth embodiment. The contents illustrated in each stage of Fig. 15 are the same as those described with reference to Fig. 3. As illustrated in Fig. 14, in step S108C, the target image acquisition unit 291C only detects Wn (= tn) and does not calculate Δtn. In addition, the target image acquisition unit 291C does not execute steps S110 and S112 described with reference to Fig. 2. Further, in step S114C, the target image acquisition unit 291C acquires the angiographic image Vm by causing the FPD to image the target blood vessel at the timing t (t = tn + Δt2) after Δt2 obtained in step S100 from the time point tn corresponding to Wn.

That is, in the fourth embodiment, the target image acquisition unit 291C does not use the "two pieces of temporally consecutive electrocardiogram data acquired immediately before" as described in the first embodiment, but sequentially acquires the angiographic image Vm at the predetermined intervals Δt2 (time interval corresponding to the pulsation cycle of the heart) calculated using "two pieces of temporally consecutive electrocardiogram data acquired at a certain time point in the past". In this way, it is possible to reduce a processing load in the control device 29C as compared with a case where Δtn is calculated every time by using the electrocardiogram data immediately before. The target image acquisition unit 291C may update the reference time interval (Δt2 in Figs. 14 and 15) for each predetermined cycle (for example, for each 10 cycles), and may acquire the angiographic image Vm using the updated time interval uniformly until the next update after the update.

As illustrated in Fig. 15, in the image correction of the second processing (Fig. 3: step S208), the image correction unit 13C generates the corrected angiographic image Vm' by the procedure of the processing a1, a2 described with reference to Figs. 5A and 5B of the first embodiment every time. To be specific, the image correction unit 13C generates the corrected angiographic image Vm' by correcting the angiographic image Vm to be corrected by moving same in parallel in such a manner that (a1) the position x2, y2 of the specific portion FP2 of the medical device included in the angiographic image Vm to be corrected approaches (a2) the position x, y of the specific portion FP of the medical device included in a single angiographic image V1 acquired temporally earlier than the angiographic image Vm to be corrected. In other words, the image correction unit 13C does not perform the processing a2' described with reference to Figs. 6A and 6B of the first embodiment.

As described above, the contents of the first processing and the second processing can be variously changed. In addition to the above-described modifications, in the first processing and the second processing, the execution order of the steps to be executed may be changed, at least a part of the steps may be omitted, and other steps which are not described may be executed. According to the surgery assistance device 10C and the angiography apparatus 20C of the fourth embodiment as described above, it is also possible to exhibit the same effects as those of the first embodiment described above. Further, according to the angiography apparatus 20C of the fourth embodiment, the predetermined intervals Δt2 are calculated from the time interval between two pieces of temporally consecutive electrocardiogram data acquired in the past by the electrocardiogram measurement device 60. Therefore, the target image acquisition unit 291C can image the angiographic image Vm in which deviation due to pulsation is accurately reduced in accordance with the pulsation cycle of the heart 91 of each patient and output the angiographic image Vm. Similarly, the angiographic image acquisition unit 12 of the surgery assistance device 10C can acquire the angiographic image Vm in which deviation due to pulsation is accurately reduced.

### <Modifications of Present Embodiments>

The present invention is not limited to the above-described embodiments, and can be implemented in various modes without departing from the scope of the invention. For example, a part of the configuration implemented by hardware may be replaced by software, and conversely, a part of the configuration implemented by software may be replaced by hardware. In addition, for example, the following modifications are also possible.

### [Modification 1]

In the first to fourth embodiments, the configurations of the surgery assistance systems 1, 1A - 1C have been exemplified. However, the configuration of the surgery assistance system 1 can be variously changed. For example, the display device 30 may be a monitor or a touch panel incorporated in the surgery assistance devices 10, 10A - 10C. For example, the angiography apparatuses 20, 20B, and 20C may have a configuration including a single FPD (in other words, a configuration not including the second FPD 25). For example, the surgery assistance system 1 may have another medical apparatus (e.g., a computerized tomography (CT) apparatus or a magnetic resonance imaging (MRI) apparatus) or the like not illustrated.

### [Modification 2]

The configurations of the surgery assistance devices 10, 10A - 10C have been described in the first to fourth embodiments. However, the configuration of the surgery assistance device 10 can be variously changed. For example, the functions of the functional units included in the surgery assistance device 10 may be implemented by cooperation of a plurality of apparatuses connected via a network.

### [Modification 3]

In the first to fourth embodiments, examples of the procedures of the first processing and the second processing have been described. However, the procedures of the first processing and the second processing described above are merely examples, and various modifications can be made. For example, the order of execution of the steps may be changed, at least some of the steps may be omitted, and other steps not described may be executed.

For example, the predetermined intervals Δtn (Δt2 in the case of the fourth embodiment) that determines the timing t in the first processing may be a mean time interval (or a time interval obtained by statistics, such as a most frequent time interval) calculated from three or more pieces of temporally consecutive electrocardiogram data acquired immediately before or in the past. In addition, the predetermined intervals Δtn (Δt2 in the case of the fourth embodiment) may be calculated from two or more pieces of electrocardiogram data which are acquired immediately before or in the past and are not temporally continuous.

For example, in step S206 of the second processing illustrated in Fig. 4, template matching may be performed using the image of the specific portion of the medical device included in the immediately preceding specific portion image. To be specific, in step S206 executed for the first time, the image correction unit 13 matches the image of the medical device included in the angiographic image V2 with the specific portion image generated using the angiographic image V1 to detect the specific portion FP2 of the medical device photographed in the angiographic image V2. Thereafter, the image correction unit 13 extracts an image of a predetermined range including the specific portion FP2 in the angiographic image V2, and updates the specific portion image in the storage unit together with the coordinate information of a center C2 (x2, y2) of the specific portion FP2. To be specific, in step S206 executed for the second time, the image correction unit 13 matches the image of the medical device included in the angiographic image V3 with the specific portion image generated using the angiographic image V2 to detect the specific portion FP3 of the medical device photographed in the angiographic image V3. Thereafter, the image correction unit 13 extracts an image of a predetermined range including the specific portion FP3 in the angiographic image V3, and updates the specific portion image in the storage unit together with the coordinate information of a center C3 (x3, y3) of the specific portion FP3. By repeating this processing, the image correction unit 13 can perform template matching using the image of the specific portion of the medical device included in the immediately preceding specific portion image. In this way, the image correction unit 13 detects the specific portion of the angiographic image Vm to be corrected by using the image of the specific portion in the angiographic image temporally closest to and immediately before the angiographic image Vm to be corrected, and thus the detection accuracy of the specific portion can be improved.

For example, in step S206 of the second processing illustrated in Fig. 4, the template matching may be performed using a specific portion pattern instead of the specific portion image. The specific portion pattern is a data group including a plurality of images obtained by imaging each of the specific portions of a plurality of medical devices used for a procedure of examination or treatment from various angles, and is prepared in advance and stored in the storage unit. In this case, in step S202, the image correction unit 13 reads the specific portion pattern, collates the specific portion FP of the medical device selected in step S202 with the images of the specific portions of the various medical devices included in the specific portion pattern, and specifies one that is actually used in the procedure. Then, in step S206, the image correction unit 13 detects the specific portion FPm of the medical device photographed in the angiographic image Vm by matching the image of the medical device photographed in the angiographic image Vm with the one datum specified in step S202.

In step S210 of the second processing illustrated in Fig. 4, the angiographic image V1 used as a reference is used in the image correction a1 in step S208 without any correction, and is output to the display device 30. However, the image correction unit 13 may correct the angiographic image V1 by translating the image in such a manner that the specific portion FP of the angiographic image V1 is located at the center of the image V1 (the corrected angiographic image V1 is also referred to as a "corrected angiographic image V1a"). In this case, the image correction unit 13 uses the corrected angiographic image V1a as a reference in the image correction a1 performed in step S208. Further, in step S210, the display control unit 14 causes the display device 30 to output the corrected angiographic image V1a. In this way, with regard to the angiographic images V1a to Vm', the specific portions FP to FPm of the images displayed on the display device 30 can be positioned at the center of the screen, and thus the operator can easily confirm the positions of the specific portions FP to FPm.

For example, in step S210 of the second processing illustrated in Fig. 4, the display control unit 14 may cause the display device 30 to display only a part (specific region image R) of the angiographic images V1 to Vm. In this case, the display control unit 14 trims (cuts out) a part (specific region image R) from the angiographic images V1 to Vm and then causes the display device 30 to display the trimmed part. For example, the specific region image R is selected together with the selection of the specific portion FP in step S202 of the second processing. In a case where only the specific region image R is displayed on the display device 30, the image correction unit 13 may translate the trimming range (cutout range) of the image instead of translating the image in step S208.

### [Modification 4]

The configurations of the surgery assistance devices 10, 10A - 10C of the first to fourth embodiments, the configurations of the angiography apparatuses 20, 20B, and 20C, and the configurations of the first to third modifications may be combined as appropriate. For example, the surgery assistance device 10 may be configured to be capable of outputting the composite image described in the second embodiment and executing the first processing described in the third embodiment. For example, in the surgery assistance system 1C that performs the first processing and the second processing described in the fourth embodiment, the output of the composite image described in the second embodiment may be enabled. For example, in the surgery assistance systems 1A - 1C described in the second to fourth embodiments, the modification of the step S206, the modification of the step S208, or the modification of the step S210 described in the third modification may be adopted.

The present mode has been described above on the basis of the embodiments and the modifications. However, the embodiment of the above-described mode is intended to facilitate understanding of the present mode and does not limit the present mode. The present mode can be modified and improved without departing from the gist and the scope of the claims, and the present mode includes equivalents thereof. In addition, if the technical features are not described as essential in the present specification, the technical features may be appropriately deleted.

### DESCRIPTION OF REFERENCE NUMERALS

1, 1A - 1C ... SURGERY ASSISTANCE SYSTEM
10, 10A - 10C ... SURGERY ASSISTANCE DEVICE
11 ... MAIN CONTROL UNIT
12, 12A, 12B ... ANGIOGRAPHIC IMAGE ACQUISITION UNIT
13, 13A, 13C ... IMAGE CORRECTION UNIT
14 ... DISPLAY CONTROL UNIT
15 ... TRUE LUMEN INFORMATION ACQUISITION UNIT
16 ... TRUE LUMEN IMAGE GENERATION UNIT
17 ... IMAGE COMPOSITION UNIT
18 ... TARGET IMAGE ACQUISITION UNIT
19 ... ELECTROCARDIOGRAM INFORMATION ACQUISITION UNIT
20, 20B, 20C ... ANGIOGRAPHY APPARATUS
21 ... FIRST FPD
22 ... FIRST X-RAY TUBE DEVICE
23 ... FIRST C ARM
24 ... FIRST SUPPORT PORTION
25 ... SECOND FPD
26 ... SECOND X-RAY TUBE DEVICE
27 ... SECOND C ARM
28 ... SECOND SUPPORT PORTION
29, 29B, 29C ... CONTROL DEVICE
30 ... DISPLAY DEVICE
31 ... MONITOR
32 ... ARM
40 ... TABLE
41 ... BED
42 ... EXPANSION/CONTRACTION PORTION
43 ... LEG PORTION
50 ... OPERATION UNIT
60 ... ELECTROCARDIOGRAM MEASUREMENT DEVICE
90 ... HUMAN BODY
91 ... HEART
92 ... HEAD
93 ... FEET
291, 291C ... TARGET IMAGE ACQUISITION UNIT
292 ... ELECTROCARDIOGRAM INFORMATION ACQUISITION UNIT
300 ... IMAGING SENSOR
301 ... TRANSDUCER
400 ... WIRE CATHETER
500 ... GUIDE WIRE

## Claims

1. A surgery assistance device comprising:
an angiographic image acquisition unit that sets a time interval corresponding to a pulsation cycle of a heart as predetermined intervals and sequentially acquires an angiographic image representing a target blood vessel into which a medical device is inserted at the predetermined intervals; and
an image correction unit that sequentially corrects the angiographic image sequentially acquired by the angiographic image acquisition unit, the image correction unit generating a corrected angiographic image by correcting the angiographic image to be corrected in such a manner that a position of a specific portion of the medical device included in the angiographic image to be corrected approaches a position of the specific portion of the medical device included in the angiographic image acquired temporally earlier than the angiographic image to be corrected.

2. The surgery assistance device according to claim 1, wherein the angiographic image acquisition unit sequentially acquires the angiographic image for which a time interval between two pieces of temporally consecutive electrocardiogram data acquired in the past by an electrocardiogram measurement device is set as the time interval corresponding to the pulsation cycle of the heart.

3. The surgery assistance device according to claim 1, wherein the angiographic image acquisition unit sequentially acquires the angiographic image for which a time interval between two pieces of temporally consecutive electrocardiogram data acquired immediately before by an electrocardiogram measurement device is set as the time interval corresponding to the pulsation cycle of the heart.

4. The surgery assistance device according to claim 2 or 3,
wherein the electrocardiogram data is electrocardiogram waveform data,
wherein when, among the two pieces of consecutive electrocardiogram waveform data, data acquired relatively later is defined as n-th electrocardiogram waveform data and data acquired relatively earlier is defined as (n-1)-th electrocardiogram waveform data, the angiographic image acquisition unit sequentially acquires the angiographic image for which a time interval between a time point tn-1 at which a specific waveform appears in the (n-1)-th electrocardiogram waveform data and a time point tn at which the specific waveform appears in the n-th electrocardiogram waveform data is set as the time interval corresponding to the pulsation cycle of the heart.

5. The surgery assistance device according to any one of claims 1 to 4, wherein the image correction unit corrects the angiographic image to be corrected in such a manner that a position of the specific portion of the medical device included in the angiographic image to be corrected approaches a position of the specific portion of the medical device included in the corrected angiographic image obtained by correcting the angiographic image acquired immediately before the angiographic image to be corrected.

6. The surgery assistance device according to any one of claims 1 to 5, further comprising:
an electrocardiogram information acquisition unit that acquires electrocardiogram data from an electrocardiogram measurement device; and
a target image acquisition unit that extracts the angiographic image at the predetermined intervals from continuous angiographic images representing the target blood vessel continuously imaged by a flat panel detector (FPD) at intervals shorter than the predetermined intervals.

7. The surgery assistance device according to any one of claims 1 to 6 further comprising:
a true lumen information acquisition unit that acquires three dimensional position information of a true lumen existing in the target blood vessel;
a true lumen image generation unit that generates a true lumen image representing the true lumen; and
an image composition unit that generates a composite image by compositing the corrected angiographic image and the true lumen image, and outputs the composite image,
wherein the true lumen image generation unit acquires, from the image correction unit, an angiographic image which is obtained by imaging the target blood vessel by an FPD disposed at a freely-selected imaging position and which is the corrected angiographic image for the angiographic image at the predetermined intervals, and generates a true lumen image representing the true lumen at a position and posture corresponding to the corrected angiographic image by using position information of the freely-selected imaging position and three dimensional position information of the true lumen.

8. The surgery assistance device according to claim 7,
wherein the angiographic image acquisition unit sequentially acquires a first angiographic image obtained by imaging by the FPD disposed at a first position and a second angiographic image obtained by imaging by the FPD disposed at a second position different from the first position, which are the angiographic image at the predetermined intervals,
wherein the image correction unit sequentially generates a first corrected angiographic image that is the corrected angiographic image for the first angiographic image, and a second corrected angiographic image that is the corrected angiographic image for the second angiographic image, and
wherein the true lumen information acquisition unit acquires three dimensional position information of the true lumen by using an ultrasonic image which is obtained by imaging an inside of the target blood vessel by an ultrasonic sensor and acquired at the predetermined intervals, position information of the first position, the first corrected angiographic image, position information of the second position, and the second corrected angiographic image.

9. A control method for controlling a surgery assistance device comprising:
an angiographic image acquisition process that sets a time interval corresponding to a pulsation cycle of a heart as predetermined intervals and sequentially acquires an angiographic image representing a target blood vessel into which a medical device is inserted at the predetermined intervals; and
an image correction process that sequentially corrects the angiographic image sequentially acquired by the angiographic image acquisition process, the image correction process generating a corrected angiographic image by correcting the angiographic image to be corrected in such a manner that a position of a specific portion of the medical device included in the angiographic image to be corrected approaches a position of the specific portion of the medical device included in the angiographic image acquired temporally earlier than the angiographic image to be corrected.

10. A computer program executed in a surgery assistance device comprising:
an angiographic image acquisition step that sets a time interval corresponding to a pulsation cycle of a heart as predetermined intervals and sequentially acquires an angiographic image representing a target blood vessel into which a medical device is inserted at the predetermined intervals; and
an image correction step that sequentially corrects the angiographic image sequentially acquired by the angiographic image acquisition step, the image correction step generating a corrected angiographic image by correcting the angiographic image to be corrected in such a manner that a position of a specific portion of the medical device included in the angiographic image to be corrected approaches a position of the specific portion of the medical device included in the angiographic image acquired temporally earlier than the angiographic image to be corrected.

11. An angiography apparatus comprising:
an FPD having an X-ray tube device and an X-ray flat panel detector; and
a target image acquisition unit that acquires an angiographic image representing a target blood vessel by causing the FPD to image the target blood vessel into which a medical device is inserted at predetermined intervals that are a time interval corresponding to a pulsation cycle of a heart, and outputs the acquired image.

12. The angiography apparatus according to claim 11, further comprising an electrocardiogram information acquisition unit that acquires electrocardiogram data from an electrocardiogram measurement device, wherein the target image acquisition unit sets a time interval between two pieces of temporally consecutive electrocardiogram data acquired in the past by the electrocardiogram information acquisition unit as the predetermined intervals that are the time interval corresponding to the pulsation cycle of the heart.

13. The angiography apparatus according to claim 11, further comprising an electrocardiogram information acquisition unit that acquires electrocardiogram data from an electrocardiogram measurement device, wherein the target image acquisition unit sets a time interval between two pieces of temporally consecutive electrocardiogram data acquired immediately before by the electrocardiogram information acquisition unit as the predetermined intervals that are the time interval corresponding to the pulsation cycle of the heart.

14. The angiography apparatus according to claim 12 or 13,
wherein the electrocardiogram data is electrocardiogram waveform data,
wherein when, among the two pieces of consecutive electrocardiogram waveform data, data acquired relatively later is defined as n-th electrocardiogram waveform data and data acquired relatively earlier is defined as (n-1)-th electrocardiogram waveform data, the target image acquisition unit sets a time interval between a time point tn-1 at which a specific waveform appears in the (n-1)-th electrocardiogram waveform data and a time point tn at which the specific waveform appears in the n-th electrocardiogram waveform data as the predetermined intervals that are the time interval corresponding to the pulsation cycle of the heart.

15. A control method for controlling an angiography apparatus comprising a target image acquisition process that acquires an angiographic image representing a target blood vessel by imaging the target blood vessel into which a medical device is inserted at predetermined intervals that are a time interval corresponding to a pulsation cycle of a heart, and outputs the acquired image.

16. A computer program executed in an angiography apparatus comprising a target image acquisition step that acquires an angiographic image representing a target blood vessel by imaging the target blood vessel into which a medical device is inserted at predetermined intervals that are a time interval corresponding to a pulsation cycle of a heart, and outputs the acquired image.

17. A surgery assistance system comprising:
the surgery assistance device according to any one of claims 1 to 5; and
the angiography apparatus according to any one of claims 11 to 14,
wherein the target image acquisition unit of the angiography apparatus sequentially transmits the angiographic image at the predetermined intervals to the surgery assistance device,
wherein the angiographic image acquisition unit of the surgery assistance device sequentially acquires the angiographic image at the predetermined intervals from the angiography apparatus, and
wherein the image correction unit of the surgery assistance device sets a latest angiographic image among a plurality of angiographic images acquired by the angiographic image acquisition unit as the angiographic image to be corrected.

18. The surgery assistance system according to claim 17, the surgery assistance device further comprising:
a true lumen information acquisition unit that acquires three dimensional position information of a true lumen existing in the target blood vessel;
a true lumen image generation unit that generates a true lumen image representing the true lumen; and
an image composition unit that generates a composite image by compositing the corrected angiographic image and the true lumen image, and outputs the composite image,
wherein the true lumen image generation unit acquires, from the image correction unit, an angiographic image which is obtained by imaging the target blood vessel by the FPD disposed at a freely-selected imaging position and which is the corrected angiographic image for the angiographic image at the predetermined intervals, and generates a true lumen image representing the true lumen at a position and posture corresponding to the corrected angiographic image by using position information of the freely-selected imaging position and three dimensional position information of the true lumen.

19. The surgery assistance system according to claim 18,
wherein the angiographic image acquisition unit sequentially acquires a first angiographic image obtained by imaging by the FPD disposed at a first position and a second angiographic image obtained by imaging by the FPD disposed at a second position different from the first position, which are the angiographic image at the predetermined intervals,
wherein the image correction unit sequentially generates a first corrected angiographic image that is the corrected angiographic image for the first angiographic image, and a second corrected angiographic image that is the corrected angiographic image for the second angiographic image, and
wherein the true lumen information acquisition unit acquires three dimensional position information of the true lumen by using an ultrasonic image which is obtained by imaging an inside of the target blood vessel by an ultrasonic sensor and acquired at the predetermined intervals, position information of the first position, the first corrected angiographic image, position information of the second position, and the second corrected angiographic image.

20. A control method for controlling a surgery assistance system comprising the surgery assistance device according to any one of claims 1 to 5 and the angiography apparatus according to any one of claims 11 to 14,
wherein the angiography apparatus sequentially transmits the angiographic image at the predetermined intervals to the surgery assistance device,
wherein the surgery assistance device sequentially acquires the angiographic image at the predetermined intervals from the angiography apparatus, and
wherein the surgery assistance device sets a latest angiographic image among a plurality of the acquired angiographic images as the angiographic image to be corrected.

21. A computer program executed in a surgery assistance system comprising the surgery assistance device according to any one of claims 1 to 5 and the angiography apparatus according to any one of claims 11 to 14,
wherein the angiography apparatus sequentially transmits the angiographic image at the predetermined intervals to the surgery assistance device,
wherein the surgery assistance device sequentially acquires the angiographic image at the predetermined intervals from the angiography apparatus, and
wherein the surgery assistance device sets a latest angiographic image among a plurality of the acquired angiographic images as the angiographic image to be corrected.
